# EUROPEAN PATENT SPECIFICATION

(11) **EP 1 593 386 B1**
(45) Date of publication and mention of the grant of the patent: **21.02.2007**
(21) Application number: 04007345.4
(22) Date of filing: 26.03.2004
(51) Int. Cl.: A61K 38/17, A61P 37/06

(54) **Use of galectin-2**
Verwendung von Galectin-2
Utilisation de galectin-2

(43) Date of publication of application: 09.11.2005
(73) Proprietor: Charité - Universitätsmedizin Berlin, 10117 Berlin (DE)
(72) Inventor: Dignass, Axel, 13158 Berlin (DE); Rosewicz, Stefan, 13465 Berlin (DE); Sturm, Andreas, 14167 Berlin (DE)
(74) Representative: Engelhard, Markus

(56) References cited:
- US-A1- 2004 023 855
- DATABASE WPI Section Ch, Week 200335 Derwent Publications Ltd., London, GB; Class B04, AN 2003-367092 XP002292305 & WO 02/089831 A1 (PROTEGENE KK) 14 November 2002 (2002-11-14)
- "GALECTINS: A FAMILY OF ANIMAL SS-GALACTOSIDE-BINDING LECTINS" CELL, CELL PRESS, CAMBRIDGE, NA, US, vol. 76, 25 February 1994 (1994-02-25), pages 597-598, XP002922187 ISSN: 0092-8674
- LIU FU-TONG: "Galectins: A new family of regulators of inflammation" CLINICAL IMMUNOLOGY (ORLANDO), vol. 97, no. 2, November 2000 (2000-11), pages 79-88, XP001182659 ISSN: 1521-6616

### SEQUENCE LISTING

<110> Charite
<120> Uses of Galectin-2
<130> FB13465
<160> 2
<170> Patent In version 3.2
<210> 1
   <211> 132
   <212> PRT
   <213> Homo sapiens
<400> 1
   Met Thr Gly Glu Leu Glu Val Lys Asn Met Asp Met Lys Pro Gly Ser
   1 5 10 15
   Thr Leu Lys Ile Thr Gly Ser Ile Ala Asp Gly Thr Asp Gly Phe Val
   20 25 30
   Ile Asn Leu Gly Gln Gly Thr Asp Lys Leu Asn Leu His Phe Asn Pro
   35 40 45
   Arg Phe Ser Glu Ser Thr Ile Val Cys Asn Ser Leu Asp Gly Ser Asn
   50 55 60
   Trp Gly Gln Glu Gln Arg Glu Asp His Leu Cys Phe Ser Pro Gly Ser
   65 70 75 80
   Glu Val Lys Phe Thr Val Thr Phe Glu Ser Asp Lys Phe Lys Val Lys
   85 90 95
   Leu Pro Asp Gly His Glu Leu Thr Phe Pro Asn Arg Leu Gly His Ser
   100 105 110
   His Leu Ser Tyr Leu Ser Val Arg Gly Gly Phe Asn Met Ser Ser Phe
   115 120 125
   Lys Leu Lys Glu
   130
<210> 2
   <211> 130
   <212> PRT
   <213> Rattus norvegicus
<400> 2
   Met Ser Glu Lys Phe Glu Val Thr Asn Leu Asn Met Lys Ser Gly Met
   1               5                   10                  15
   Ser Leu Lys Ile Lys Gly Lys Ile His Asn Asp Val Asn Ser Phe Thr
               20                  25                  30
   Ile Asn Leu Gly Gln Gly Lys Glu Thr Leu Asn Leu His Phe Asn Pro
           35                  40                  45
   Arg Phe Asn Glu Ser Thr Ile Val Cys Asn Thr Leu Asp Gly Ser Ser
       50                  55                  60
   Trp Gly Gln Glu Gln Arg Glu Asn His Ser Cys Phe Ser Pro Gly Ser
   65                  70                  75                  80
   Glu Val Lys Leu Thr Leu Thr Phe Gln Asp Lys Asp Phe Lys Val Thr
                   85                  90                  95
   Leu Pro Asp Gly His Ser Leu Thr Phe Pro Asn Arg Leu Gly His Asn
               100                 105                 110
   His Leu Arg Tyr Leu Ser Met Asp Gly Leu Gln Ile Ser Ser Phe Lys
           115                 120                 125
   Leu Glu
       130

## Description

The present invention relates to uses of galectin-2.

The central function of the immune system of higher animals is the distinction between foreign entities, such as infectious agents, bacteria, viruses etc., from self components of the body. The immune systems is a dynamic system showing a plastic behaviour which means that the immune system is a learning system throughout the entire life span of an individual. During the development of the body, usually, the immune system acquires the ability to tolerate self components whilst developing and maintaining the capability of recognizing foreign components. The acquisition of self tolerance occurs by clonal deletion of autoreactive T-cells by apoptosis in the thymus gland during the perinatal period, and by functionally suppressing autoreactive T and B cells at later stages of the development. At present, it is not entirely clear how the system of self tolerance is maintained or how, under specific circumstances, it breaks down. Sometimes the organism loses its capability of self tolerance and the organism's immune system fails to discriminate between self and non-self antigens, as a result of which an autoimmune response is launched which manifests itself in the activation and clonal expansion of autoreactive lymphocytes and the production of auto-antibodies against autologous antigens of normal body tissues. There exist different animal models for various autoimmune diseases, but from these it is not clear what causes the onset of an autoimmune response nor, in many cases, what the further course of events after an initial autoimmune response will be. Many of the autoimmune diseases are rare. Multifactorial in origin, autoimmune diseases occur more frequently in individuals with a genetic pre-disposition. They may be caused by specific weaknesses of immune regulatory controls, certain environmental factors, etc. the combination of which may ultimately lead to the immune system launching an attack against self components.

Autoimmune diseases can be classified into preferably systemic and preferably organ-specific diseases. In systemic autoimmune diseases tissue injury and inflammation occurs in multiple sites in organs without relation to their antigenic make up. In many instances such systemic autoimmune diseases are initiated by the deposition of circulating autologous immune complexes which are formed by auto-antibody responses to ubiquitous soluble cellular antigens. Typical examples of these systemic autoimmune diseases are systemic lupus erythematodes, scleroderma, polymyositis, rheumatoid arthritis and ankylosing spondylitis. Organ-specific autoimmune diseases, on the other hand, can be further classified according to the organ they affect. Autoimmune diseases of the nervous system include multiple sclerosis, myasthenia gravis, autoimmune neuropathies, such as Guillain-Barré. Autoimmune diseases affecting the gastrointestinal system are Crohn's disease, ulcerative colitis, primary biliary cirrhosis, primary sclerosing cholangitis, sprue's disease, auto-immune enteropathy, and autoimmune hepatitis. Autoimmune diseases effecting endocrine glands are diabetes mellitus type 1, Grave's disease, Hashimoto's thyroiditis and autoimmune oophoritis.

Crohn's disease (CD) is an autoimmune disease which can affect any part of the digestive tract, from the mouth to the anus. However, it usually occurs in the lower part of the small intestine. It manifest itself in a severe inflammation extending deep into the lining of the intestine. The symptoms of Crohn's disease are abdominal pain, diarrhea, rectal bleeding, weight loss and fever. The etiology of Crohn's disease remains unclear. Diagnosis usually is made by the presentation of typical clinical symptoms, and a combination of blood tests, colonoscopy including biopsies, x-ray examinations with contrasting agents, magnetic resonance imaging or CT. Treatment of Crohn's disease includes anti-inflammatory agents such as mesalazine, sulfasalazine, corticosteroids and immunosuppressants. The latter include 6-mercaptopurine and azathioprine. Recently a monoclonal antibody (mAb) infliximab has been authorized for the treatment of moderate to severe or fistulizing Crohn's disease which does not respond to standard therapies, such as mesalasazine, corticosteroids and/or immunosuppressive agents and antibiotics in fistulizing CD. Infliximab which is the first agent approved specifically for RA and Crohn's disease is a chimeric antibody against tumor necrosis factor α (anti-TNF α). TNF α is a cytokine produced by the immune system and plays a crucial role in the pathogenesis of Crohn's disease. Treatment of patients with CD induces apoptosis of lamina propria T-cells and macrophages located in the intestinal mucosa or joints. However, during treatment with infliximab, frequently other antibodies are generated which attenuate the action of the anti-TNF α-antibody (New England Journal of Medicine 2003; 348:601-608) and cause intolerances (type I hypersensitivity). Furthermore, infliximab appears to suppress the immune system in general, thus exposing the organism to secondary super infections and therefore causing sepsis, tuberculosis and death.

Rheumatoid arthritis (RA) is also believed to be an autoimmune disease. Rheumatoid arthritis causes inflammation and deformity of the joints. Other problems throughout the body may also develop, including inflammation of blood vessels, the development of rheumatoid arthritic nodules and osteoporosis. Rheumatoid arthritis is considered an autoimmune disease which is acquired and in which genetic factors appear to play a role. The presence of HLA-DR4 antibody in 70 percent of patients with RA lends support to the genetic predisposition to the disease. Patients having rheumatoid arthritis, in many instances also show rheumatoid factors (RF), which are antibodies to IgG and which are present in 60-80 percent of adults with the disease. High titers of rheumatoid factors are usually associated with more severe and active joint disease, greater systemic involvement and a poorer prognosis for remission. Rheumatoid arthritis, as well as other autoimmune diseases, includes widespread immunologic and inflammatory alterations of connective tissue. Because the autoimmune diseases share many clinical findings, making a differential diagnosis is often difficult. The prevalence of the disease is 1-2 percent of the general population and is found world-wide. There seems to be a certain pre-disposition in women which show a 3:1 ratio to men with respect to the occurrence and onset of the disease. The disease usually manifests itself in adults between the age of 40 to 60 years. The etiology of rheumatoid arthritis remains unknown. Metabolic and nutritional factors, the endocrine system, geographic, psychologic and occupational data have been extensively studied with no conclusive findings. There is a number of etiological theories on rheumatoid arthritis, some of which postulate an unknown antigen which initiates the autoimmune response. Others suspect an infectious origin of the disease process, which has included various bacteria and viruses but without evidence of precipitating events. Again, rheumatoid arthritis is diagnosed using standard criteria (ARA) (American Rheumatism Association - see also for example http://www.shim.org/rheumatology/1987ra.html) including a number of tests, including blood tests, in particular tests for the erythrocyte sedimentation rate, tests for anemia and tests for Rheumatoid factor. Furthermore, because rheumatoid arthritis in particular affects synovial joints, the synovial fluid is examined. When rheumatoid arthritis is present, the fluid has an increased protein content and a decreased or normal glucose content. Furthermore it contains a higher than normal number of white blood cells. Usually diagnosis of rheumatoid arthritis is a combination of positive results of various of the aforementioned tests. Rheumatoid arthritis can be treated in a number of ways, although there is no cure up to date. Rheumatoid arthritis may be treated by non-steroidal anti-inflammatory agents, by anti-rheumatic drugs, including penicillamine and cyclo-oxygenase-2-inhibitors. Furthermore, like Crohn's disease, rheumatoid arthritis is also being treated by antibodies directed against TNF α, such as infliximab, ethanercept and adalimumab. Additionally, quite recently an IL-1 receptor antagonist, anakinra, has been market authorized for the treatment of RA. According to preliminary studies, anakinra is suggested to be administered in combination with methotrexate which, again, may have severe side effects. However, all of the aforementioned agents show more or less severe side-effects.

Accordingly, there exists a need in the art for alternative and efficient treatments for autoimmune diseases. In particular there exists a continuing need in the art for an efficient treatment for inflammatory bowel diseases and rheumatoid arthritis.

Galectins are members of a highly conserved family of β-galactoside binding lectins that more and more emerge as significant regulators of immune cell homeostasis (1, 2). The biological properties of mammalian galectins include regulation of cell proliferation, inflammation, cell adhesion and cell death (3-5). Interestingly, despite extensive sequence homology and similar carbohydrate specificity, various members of this protein family behave as amplifiers of the inflammatory cascade, while others activate homeostatic signals to prevent immune responses (6). Hence, what applies to one member of the galectin-family, does not necessarily apply to other members as well.

Galectin-2 was discovered during the cloning of galectin-1 and has 43% sequence identity to human galectin-1, revealing the greatest homology with galectin-1 among all others galectins examined (7). Galectin-2 is a non-covalent dimer with subunits of about 14 kDa (7). Expression of galectin-2 seems to be restricted to the gastrointestinal (GI) tract (8, 9). It is also known that intestinal epithelial cells that express galectin-2 do not normally express galectin-1 and that galectin-2 is not expressed at elevated levels in galectin-1 null mutant mice (10). Whereas the properties of galectin-1 have been studied more extensively, the function of galectin-2 has not been studied so far and thus remains unclear until now.

The ability of cells to adhere to constituents of the extracellular matrix (ECM) is a fundamental process involved not only in cell growth, apoptosis, angiogenesis, tumor invasion and inflammation (14, 15). Adhesion of T cells to the ECM is mediated by integrins, a large family of heterodimeric receptors consisting of eight different β subunits and 17 α chains that control matrix ligand specificity (16, 17). Galectins modulate cell adhesion by a different mechanism including bridging carbohydrate ligands on neighbouring cells, cross-linking galectins that are associated with the cell surface, direct binding to ECM compounds, protein-carbohydrate interactions or interaction with integrins (14, 18). The manifold options of galectins to influence cell-cell and cell-ECM adhesion may explain why galectins have pro- and anti-adhesive effects in different cell types and with distinct ECM constituents (6). Circulating blood T cells express only moderate amounts of β₁ integrins on their cell surface, yet these resting cells adhere poorly to ECM (17). Activation of blood T cells stimulates a rapid augmentation in β₁-mediated adhesion associated with enhanced integrin affinity and avidity (17), required for the migration of immune cells and homing to effector sites in the integrated human mucosal immune system (19). Galectin-1 binds to β1 integrin and transiently increases its availability on the cell surface whereas galectin-3 mediates the endocytosis of and thus down-regulation of β1 integrins in breast carcinoma cells (18, 20).

US-Patent Application US 2004/0023855 A1 disposes particles for use in biological systems for delivering biologically active agents to cells or tissues. The application discloses, amongst others, as bioactive components galectins. Galectins in general are disclosed as playing a role in inflammatory, immunomodulatory and tumorigenic processes and furthermore it discloses various galectins having a diverse range of biological activities. An activity of galectin-2 is not disclosed.

JP 20010129200 discloses the usefulness of galectin-1, 3 and 9 as effective ingredients for prevention and treatment of nephritis. Galectin-2 is not discussed nor is any activity thereof disclosed.

Immune cells must respond to antigens in a selective and balanced fashion that allows them to mount an effective response by progressing through the cell cycle, expanding and finally undergoing apoptosis once the antigen has been cleared (11, 12). Whereas this feature is usually tightly balanced by promoters and inhibitors of cell cycling and apoptosis (13), many diseases are based on either uncontrolled cell cycling or impaired apoptosis, leading to unrestrained cell proliferation and thus cancer or auto-immune disorders such as rheumatoid arthritis or inflammatory bowel diseases.

These diseases are up to date not curable, and existing treatments are either not very efficient or they show severe side effects.

Accordingly, it was an object of the present invention to provide for an efficient treatment of diseases with a malfunctioning immune system. It was furthermore an object of the present invention to provide for a treatment which shows fewer side effects than methods presently available. It was also an object to provide for an efficient treatment of diseases with an impaired apoptosis of T-cells.

All these objects are solved by the use of galectin-2 or of a nucleic acid coding for galectin-2 or of its complementary strand, or a nucleic acid hybridizing to such coding nucleic acid or its complementary strand, for the manufacture of a medicament for the treatment of a patient having a disease with impaired apoptosis of T-cells.

In one embodiment said impaired apoptosis, in particular said impaired apoptosis of T-cells is involved in or associated with the pathogenesis of said disease.

Said disease with impaired apoptosis of T-cells is selected from the group comprising autoimmune diseases and malignant T-cell diseases. Preferably said autoimmune diseases are selected from the group comprising rheumatoid arthritis, inflammatory bowel diseases, multiple sclerosis, psoriasis, lupus erythematodes, scleroderma, autoimmune hepatitis and autoimmune nephritis, wherein, more preferably, said inflammatory bowel diseases are Crohn's disease or colitis ulcerosa or indeterminate colitis.

Preferably said inflammatory bowel disease is Crohn's disease.

In another embodiment, said autoimmune disease is rheumatoid arthritis.

In one embodiment said malignant T-cell diseases are selected from the group comprising peripheral and lymphoblastic/nodal and extranodal T-non-Hodgkin-lymphomas.

Preferably said galectin-2 is human or rat galectin-2.

In one embodiment said galectin-2 has an amino acid sequence selected from the group comprising SEQ ID NO: and SEQ ID NO: 2.

In one embodiment said galectin-2 is administered in combination with an agent suppressing T-cell proliferation and/or an agent inducing T-cell apoptosis.

Preferably said agent suppressing T-cell proliferation is selected from the group comprising steroids, macrolides, such as cyclosporin and rapamycin, tacrolimus, azathioprine, 6-mercaptopurine, methotrexate and cyclophosphamide.

Preferably said T-cell apoptosis inducing agent is selected from the group comprising anti-TNFα-antibody (infliximab, adalimumab and CDP 870), etanercept, leflunamide, natalizumab (anti-Integrin α4β7 mAb), visilizumab (anti-CD3 mAb).

In one embodiment said galectin-2 is administered in combination with a drug that induces T-cell apoptosis via a caspase-8 dependent pathway, wherein such drug may be one of the aforementioned ones.

In one embodiment said galectin-2 is administered in a patient failing or having failed to show a measurable response to a drug which is known to normally induce T-cell apoptosis via a caspase-8 dependent pathway, wherein such a drug may be one of the aforementioned ones.

In one embodiment said galectin-2 is administered in combination with anti-inflammatory drugs such as 5-Aminosalicylates (5-ASA), mesalazine, olsalazin, balsalazin, sulfapyridin and non-steroidal anti-inflammatory agent and/or an antirheumatic agent, wherein, preferably, said antirheumatic agent is a disease modifying anti rheumatic drug (DMARD) wherein, more preferably, said disease modifying anti-rheumatic drug is selected from the group comprising aspirin, naproxen, diclofenac, ibuprofen, naprosyn, indomethacin, piroxican and biological drugs selected from the group comprising anakinra and etodolac.

In one embodiment, said antirheumatic agent is selected from the group comprising gold compounds, D-penicillamin, antimalaria drugs such as chloroquin, and sulfasalazine.

Preferably, said galectin-2 is administered in combination with cyclo-oxygenase-2-inhibitors (COX-2-inhibitors), wherein, preferably, said cyclo-oxygenase-2-inhibitors are selected from the group comprising celecoxib, rofecoxib, and valdecoxib.

In one embodiment, said galectin-2 is administered in combination with a T-cell activating agent.

In one embodiment, said galectin-2 is administered in combination with a β-galactoside, wherein, preferably, said β-galactoside is lactose.

In one embodiment, said galectin-2 is administered by systemical administration and/ or topical administration, wherein, preferably, said administration occurs by ingestion, preferably orally or anally, and/or by injection, preferably by intravenous, intramuscular, intraperitoneal or subcutaneous injection, and/or by nasal application.

In one embodiment, said galectin-2 is administered as enema and/or as suppository and/or as delayed release dosage form, e. g. encapsulated in a pH dependent release matrix.

In one embodiment said galectin-2 is administered in a pegylated or non-pegylated form or as a mixture of the forms.

In one embodiment, said patient is one having a pathological condition in which, before administration of galectin-2, a subset of the patient's T-cells and/or a subset of the patient's macrophages and/or a subset of the patient's antigen-presenting-cells fail to undergo apoptosis, preferably adequate apoptosis or wherein a subset of the patient's T-cells and/or macrophages and/or antigen-presenting cells show an impaired or defective apoptosis.

In one embodiment, said subset of T-cells are T-cells that have previously been activated, preferably via the CD3-pathway or the CD2-pathway or via mitogens, co-stimulatory molecules such as CD28 or CD40, or other pathways such as Toll-like receptors or integrins.

In one embodiment, said subset of T-cells and/or macrophages and/or antigen-presenting cells are not resting.

In one embodiment, said subset of T-cells and/or macrophages and/or antigen-presenting cells are not cells that have exited from the cell cycle or are not cells that are arrested in any phase of the cell-cycle.

Preferably said subset of T-cells and/or macrophages and/or antigen-presenting cells is primarily located in said patient's joints, preferably synovial joints, and/or in said patient's gastrointestinal tract, preferably the lining of said gastrointestinal tract, and/or in said patient's skin, and/or lung and/or liver and/or kidney and/or are a population of peripheral blood cells which are recruited in a mucosa during inflammation.

Preferably said patient is one having a pathological condition in which, before administration of galectin-2, the ratio between Bcl-2-protein and Bax-protein in T-cells is disbalanced in favour of the anti-apoptotic Bcl-2.

The objects of the present invention are furthermore solved by the use of galectin-2 or of a nucleic acid coding for galectin-2 or of its complementary strand, or of a nucleic acid hybriding to such coding nucleic acid or to its complementary strand, as an immunomodulating agent, wherein, preferably, galectin-2 acts on T-cells, wherein, more preferably, said T-cells are human.

As used herein, the term "galectin-2" is meant to designate a protein as encoded by SEQ ID NO: 1 or SEQ ID NO: 2 and proteins having more than 90% sequence identity thereto (i. e. over 90% of the entire length of SEQ ID NO: 1 or SEQ ID NO: 2). It also is meant to signify any functional variant thereof which shows a selective induction of T-cell apoptosis in activated T-cells.

As used herein, the term "a disease with impaired apoptosis of T-cells" is meant to signify any disease which is accompanied by and/or caused by an impaired apopotis of T-cells.

The term "involved in or associated with the pathogenesis of said disease", as used herein is meant to signify that the impaired apoptosis may, but does not necessarily have to be the cause for said disease. In a preferred embodiment, said impaired apoptosis is one of the causes for said diseases, albeit not necessarily the only one.

The term "impaired apoptosis of T-cells" is meant to designate the notion that T-cells having served their function of eliminating an antigen, in the normal course of events (i. e. the non-pathological course of events) should undergo apoptosis, i. e. a programmed self destruction, whereas in a pathological condition, such as a disease, and where they show "impaired apoptosis" they do not undergo apoptosis at all or at a slower rate than normal.

The term "T-cell proliferation" is meant to signify the process in which T-cells undergo a cell-cycle of G1, S, G2 and M-phases, as a result of which the cell is divided into two cells at the end of the cycle.

The term "galectin-2 is administered ...", as used herein, is meant to signify the it may be administered as a protein or as the nucleic acid encoding galectin-2-protein and/or as the nucleic acid strand complementary to the coding nucleic acid. In a preferred embodiment, it is meant to signify that galectin-2 be administered as a protein.

The term "... is administered in combination with ...", as used herein is meant to signify that galectin-2 may be administered in conjunction with other agents. The administration may be simultaneous or one after the other, it may be via the same route or via different routes, it may be in the same dosage form or via separate dosage forms.

An "agent suppressing T-cell proliferation" is an agent that locks (or slows down) a T-cell anywhere in the cell cycle.

An "agent inducing T-cell apoptosis" is meant to designate any agent that causes the cell to undergo programmed cell death, which is also commonly referred to as apoptosis.

The term "T-cell activating agent", as used herein, is meant to indicate any agent capable of inducing a T-cell to enter from a resting state into a state in which the T-cell undergoes the cell cycle or at least parts of it.

Administration of galectin-2 may be in any form suitable for the recipient, provided that an uptake of galectin-2 into the recipient's organism is ensured. Administration may additionally be in combination with any pharmaceutically acceptable carrier. It may be systemic and/or topical, it may be injected, preferably intravenously, intramuscularly, intraperitoneally and/or subcutaneously, or it may be applied via other routes, e. g. as enema or suppository or via nasal application.

The inventors have surprisingly found that galectin-2 displays unique immunomodulatory properties characterized by specific binding of galectin-2 to the β1 subunit of integrins, a vigorous induction of T cell apoptosis by triggering the mitochondrial death pathway and alteration of integrin expression and cell adhesion, which makes galectin-2 particularly suitable for the treatment of diseases wherein T-cells are showing an impairment of their apoptotic behaviour. These diseases are autoimmune diseases or malignant diseases, in particular malignant T-cell diseases. All peripheral and lymphoblastic/nodal and extranodal T-non-Hodgkin-Lymphomas show significant expression of β1 integrin chains. It can therefore be assumed that this increased surface expression of β1 integrin influences the behavior of the malignant cell. Gal-2 down-regulates β1 expression in T-cells and may thus prevent attachment of malignant cells to the extracellular matrix. Therefore, Gal-2 may possibly have positive effects in the treatment of malignant T-cell diseases.

Galectin-2, in particular human galectin-2, is not immunogenic and therefore is particularly well suited for application as a medicament.

T cell function is determined by its activation and subsequent proliferation, cytokine secretion, but also termination of its action by apoptosis. Disturbances of this tightly balanced interplay are observed in many diseases, including inflammatory bowel disease (42). Galectin-1 has recently been shown to ameliorate experimentally induced colitis and hepatitis in mice by inducing T cell apoptosis (27, 43), whereas galectin-3 revealed anti-apoptotic properties in diverse cell populations (44). Thus, conclusions with respect to one galectin-family member cannot be transferred to another galectin-family member.

The expression, binding and biological function of galectin-2 was unclear until now, and the experiments described below show for the first time that galectin-2 has powerful immunomodulatory effects in human T cells, clearly distinct from other galectin family members.

In the following, the results of the experiments leading to the present invention are summarized, outlined and shortly discussed:

In contrast to galectin-1 and -3 (6), the present inventors found that galectin-2 is not expressed in resting or activated T cells, independent of the origin as naïve peripheral blood or tissue bound memory T cell. This finding is in accordance with the restricted galectin-2 mRNA expression found only in gastrointestinal epithelial cells (9). By immunohistochemistry the present inventors observed Gal-2 expression in colonic epithelial cells in specimen from patients with Crohn's disease and ulcerative colitis.

Galectins are carbohydrate-binding proteins that cross-link β-galactoside-containing cell surface glycoconjugates, resulting in the modulation of cell function (45). Our data show that galectin-2 binds to T cells within hours and remains bound for more than 72 hours. This binding was inhibited by lactose, indicating that galectin-carbohydrate interactions are responsible for galectin-2 binding to T cells. Comparable to galectin-1 (25), binding of galectin-2 to T cells was comparable in resting anti-CD3 or PMA/PHA (phorbol myristate acetate (PMA); phytohemagglutinin (PHA) stimulated T cells, demonstrating that galectin-2 binding to T cells is independent of their activation status.

The ability of galectin-2 to bind to T cells was significantly reduced by blocking integrin-β1 mAbs, identifying β1 as a candidate glycoprotein receptor that mediates binding of galectin-2 on the T cell surface. Galectin-1 interacts also with the β1 subunit of integrins (18), underscoring the important role of integrin-β1 in galectin binding. However, in contrast to galectin-1, galectin-2 binding to PBT could not be prevented by CD3 or CD7 mAb, indicating that both galectins have different binding sites on T cells (24). Immuno-precipitation using galectin-1 and galectin-2 coated magnetic beads confirmed the interaction of galectin-1 with the CD3, CD7 and β1 molecule (18, 24). In contrast with galectin-1, but in accordance with the blocking experiments, in which neither CD3 nor CD7 bound to galectin-2, the β1 subunit of integrins co-immunoprecipitated with galectin-2, indicating that the capability of β1 to inhibit galectin-2 binding to T cells is based on a physical interaction.

Apoptosis is a major immunomodulatory feature of T cells to terminate cell function once the antigen has been cleared, and thus preventing autoimmunity or malignancy. Furthermore, drugs that induce apoptosis of T cells or macrophages like anti-tumour necrosis factor-α, are strongly effective in treating autoimmune diseases, such as rheumatoid arthritis or inflammatory bowel disease (46, 47). Galectins studied so far have a remarkable capability to alter cell apoptosis in various cell types, e.g. galectin-1 induces and galectin-3 prevents cell death (6). However, nothing was known in this respect on galectin-2. Our data show that galectin-2 strongly induces T cell apoptosis to an extent even higher than that inducible by galectin-1. Galectin-2 required stimulation of the cells to induce apoptosis, showing that resting T cells are prevented from galectin-2 induced cell death. Galectin-2 also induced little amount of necrosis, demonstrating that galectin-2 not only induces the programmed cell death, but also kills cells by necrosis, a feature which has not previously been described for galectins at all.

The ability of lactose to suppress galectin-2 induced apoptosis indicates that β-galactoside binding activity is essentially necessary for galectin-2 induced cell death. Interestingly, since binding of galectin-2 to T cells is independent from the activation status of the cells as shown above, and since induction of apoptosis by galectin-2 requires additional cell activation, β-galactoside binding alone seems not to capable to initiate the cell death program. Galectin-2 binds to integrin β1 on the cell surface and β1 integrin mAb inhibit galectin-2 binding to T cells. However, the blocking β1 integrin mAb failed to inhibit galectin-2 induced apoptosis, suggesting that this receptor is not required for cell death. Cycloheximide was not able to prevent galectin-2 induced T cell death, indicating that in contrast to galectin-9 (28), de-novo protein synthesis seems not to be required, which was further substantiated by the fast induction of apoptosis in T cells following galectin-2 within 6-12h. It has also been reported that galectin-1 induces apoptosis distinctively in CD4⁺ and CD8⁺ positive thymocytes (38, 48, 49) and that CD4⁺ human T cell lines are more susceptible towards galectin-9 induced cell death (28). However, when splenic T cell in nephritic rats were examined, galectin-9 induced selective apoptosis of activated CD8⁺, but not CD4⁺ T cells (50). When CD4 and CD8 positive T cell subpopulations were separately examined, the rate of galectin-2 induced cell death was comparable in each cell population, demonstrating that in PBT, galectin-2 induces apoptosis independent from their status as CD4⁺ or CD8⁺ T cell.

Apoptosis is executed by different pathways, initiated by distinct caspases. The present inventors' results demonstrate clear differences in caspase activity utilized by different galectins. When PBT were activated in the presence of galectin-2, caspase-3 and -9, but not -8 activity was induced. In addition, galectin-2 mediated apoptosis was prevented by the broad spectrum caspase inhibitor zVAD, as well as by caspase-3 and -9 inhibitors, further supporting the functional relevance of caspase-3 and -9 activation in galectin-2 induced cell death. Despite the fact that caspases are the central mediators of apoptosis (29), the role of caspases in galectin induced apoptosis has been rarely examined. It has been shown that galectin-1 induces caspase-8 and -9 activity in an experimental mouse model (27), but reduces caspase-3 activity in concanvalin A-induced hepatitis in mice (43). Since caspase-3 is activated by upstream caspases such as caspase-8 or -9, these findings seem not to be in accordance and may be explained by the different models of inflammation or cell types used. The ability of galectins to induce apoptosis is not restricted to prototype galectins since the tandem-repeat galectin-9 also induces apoptosis via a calcium-calpain caspase-1 dependent pathway (28). In contrast to galectin-2, also galectin-7 induced T cell apoptosis comparable to galectin-2, however, an entirely different pattern of caspases seemed to be involved. Whereas the broad spectrum caspase inhibitor zVAD and caspase-3 inhibitor zDEVD prevented galectin-7 induced apoptosis, blocking of the upstream caspases-1 and -8, but not -9 abridged T cell death. These findings demonstrate, that galectin-2 activates a distinct cascade of caspase to execute cell death compared to other galectins.

Caspase-9 is involved in the mitochondrial pathway of apoptosis and is activated when building a complex with cytochrome c and APAF-1 (29). Cytochrome c is released from mitochondria upon disturbance of the electrochemical gradient of the inner mitochondrial membrane, a process intimately regulated by bcl-2 family members (29, 51, 52). The present inventors' data show that galectin-2 disrupts the mitochondrial membrane potential and initiates cytochrome c release, showing that the mitochondrial pathway is used by galectin-2 to induce T cell death. Bcl-2 family members meet at the surface of mitochondria, and the susceptibility of a cell towards apoptotic stimuli is dependent on the relative balance of pro- and anti-apoptotic family members (52). Galectin-2 decreased anti-apoptotic bcl-2, while increasing pro-apoptotic bax levels, resulting in a lower bcl-2/bax ratio. In autoimmune diseases such as Crohn's disease, the bcl-2/bax ratio is higher than in controls (53), and the capability of galectin-2 to lower this ratio and thus to induce cell death in uncontrolled proliferating cells, may be an appealing approach to use galectin-2 therapeutically in such diseases.

Apoptosis and cell cycle are ultimately linked and T cells in G0 are protected from antigen-induced cell death (29, 54). The requirement to cycle is also true for galectin-2 induced cell death, however, in contrast to galectin-1, which profoundly inhibits T cell proliferation (38), galectin-2 did not modulate PBT cell cycling. The data so far available studying the impact of galectins on cell cycling mostly used thymidine incorporation to measure cell proliferation (38, 55). Although simple and reproducible, this method only measures DNA synthesis during the S phase, provides no information on the fraction of cells going through other phases and does not assess for apoptotic cells, unable to cycle. However, as determined by cyclin B1 expression, a highly sensitive tool to quantify progression through the cell cycle up to the G2/M-phase (56), galectin-1 and -7, but not galectin-2 strongly suppress cell cycle progression in PBT. Furthermore, demonstrating that the incapability of galectin-2 to influence T cell cycling is not due to a shift of cell cycle promoters and inhibitors, provides evidence that the cell cycle machinery of T cells is not affected by galectin-2, a feature clearly distinct from galectin-1 and -3. The present inventors and others have shown that galectin-1 binds to the T cell receptor (TCR)(24, 38) and Vespa and coworkers have demonstrated, that galectin-1 in conjunction with anti-TCR mAbs induces apoptosis, but antagonizes anti-TCR-induced IL-2 production (38). Our observation that galectin-2 does not bind to the TCR may explain, why galectin-2 does not inhibit T cell cycling, again demonstrating that the behaviour of galectin-2 is different to that of galectin-1.

T cells continually circulate in search for foreign antigens and the recruitment and retention of T lymphocytes within inflamed tissues are dependent on adhesion to an acellular network of proteins, glycoproteins, and proteoglycans known as the extracellular matrix (ECM)(57, 58). Adhesion is a critical component of the T cell mediated immune response and is therefore tightly regulated (59-61). Galectins modulate cell adhesion by influencing direct effects such as sterically preventing the adhesion to cell-surface integrins, but also by binding to extracellular domains of one or both subunits of an integrin or internalisation of integrins (20, 39, 45). Adhesion of T cells to ECM compounds is divergently regulated by galectins and depends on the cell type and ECM protein examined (6, 14, 62). Cell adhesion to ECM compounds is mediated by the β1 family of integrins, and the present inventors have shown that galectin-2 inhibits the conformational change of the integrin subunit required to achieve high affinity. This effect can not be explained simply by unspecific binding of galectin-2 to the cell surface and thus preventing integrin activation, since i) expression of other integrins was not affected by galectin-2 and ii) in intestinal T cells, this effect was not observed. Furthermore, the upregulation of α5 by galectin-1 and -2 may suggest, that the proposed internalisation of integrins by galectins (18, 20) may not be true for all integrins. Whereas galectin-1 inhibited T cell adhesion to both, collagen and fibronectin, galectin-2 inhibited T cell adhesion to collagen type I, but in contrast to galectin-1, profoundly increased cell attachment to fibronectin. In contrast to both, galectin-1 and-2, galectin-7, did not influence T cell adhesion to collagen type I or fibronectin, indicating that galectin-2 exerts unique features not only with regard to apoptosis, but also cell adhesion. The attachment of T cells to their respective ECM compound was mediated by specific collagen and fibronectin receptors, as demonstrated by blocking experiments. It is not clear, why cell adhesion to fibronectin is promoted by galectin-2 despite the fact that galectin-2 reduces β1 integrin expression. However, regulation of cell adhesion by galectins seems to be complex, since galectin-1 has pro-adhesive properties in melanocytes, teratocarcinoma cells or fibroblasts, but inhibits T cell adhesion to laminin and fibronectin (reviewed in (6)). The same is true for galectin-3 that greatly increases the adhesion of neutrophils to various substrates (63), but blocks adhesion of tumour cell lines to laminin, fibronectin or collagen (64). Furthermore, reducing cell proliferation, as it is the case for galectin-1 (38) may explain downregulation of β1 integrin expression, but since galectin-2 downregulates β1 integrin expression without affecting cell cycling, regulation of integrin expression by galectins is independent from their ability to regulate cell proliferation. Using a large panel of blocking integrin antibodies, the inventors demonstrated that binding of T cells was mediated through distinct collagen and fibronectin receptors, and pre-incubation of T cells with β1 mAbs further inhibited T cell binding to collagen and prevented the upregulation of cell adhesion to fibronectin by galectin-2. This suggests that modulation of cell adhesion by galectin-2 is β1 integrin dependent. Furthermore, the inhibition of cell adhesion by blocking β1 mAbs was prevented by galectin-2 pre-incubation, indicating that binding of galectin-2 to T cells can functionally inactivate the β1 subunit of integrins on the cell surface.

In summary, the present inventors' studies provide clear evidence that galectin-2 binds in a carbohydrate-dependent fashion to the β1 integrin subunit, but not the TCR complex. Galectin-2 induces T cell apoptosis via the mitochondrial, intrinsic death pathway, exclusively in proliferating, but not resting T cells. The induction of T cell apoptosis in activated cells, without down-regulating cell cycling, allows a sufficient T cell response after antigen contact, and prevents unselective apoptosis of resting T cells, not being involved in the course of the specific immune response. Furthermore, by selectively regulating cell adhesion to the ECM, galectin-2 may contribute to T cell homing once the cells are activated. Thus, our data provide clear evidence that galectin-2 modulates the human immune system by mechanisms clearly distinct from other galectins, give a new insight how galectins may interact with T cells, and offers a new concept in the treatment of diseases with impaired T cell apoptosis like Cohn's disease or rheumatoid arthritis.

The invention will now be described by reference to the following specific description and the examples which are given to illustrate not to limit the present invention.

In the figures:

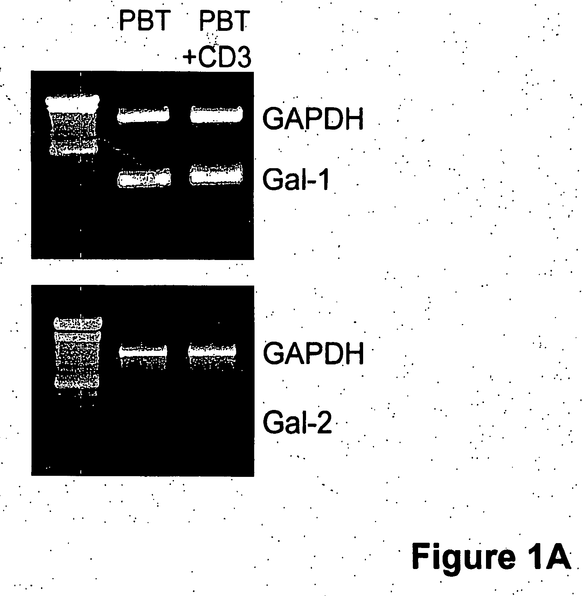

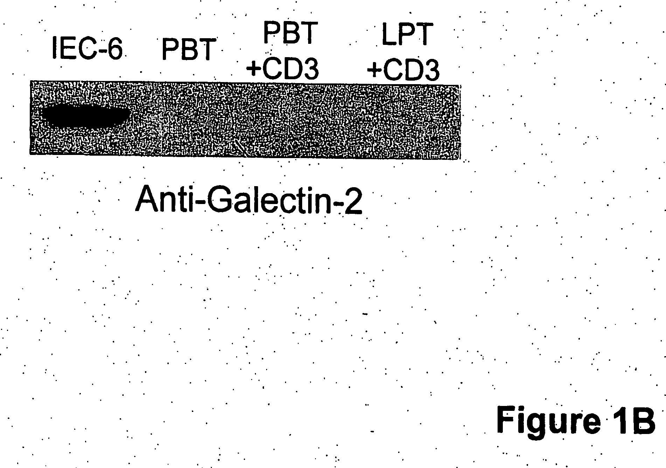

**Figure 1 shows different expression of galectin-1 and -2 in T cells.** Galectin-1 a) mRNA and b) protein expression is clearly detected in resting and activated PBT, while neither galectin-2 a) mRNA nor b) protein levels expression were detected in PBT or LPT. Cells were cultured in the absence and presence of cross-linked anti-CD3 mAb or CD2 (T11₂/T11₃) antibody for 72 hours, and mRNA and protein expression was determined by PCR analysis and western blotting, respectively. The non-transformed intestinal epithelial cell line IEC-6 served as positive control. The graphs are representative of three different experiments.

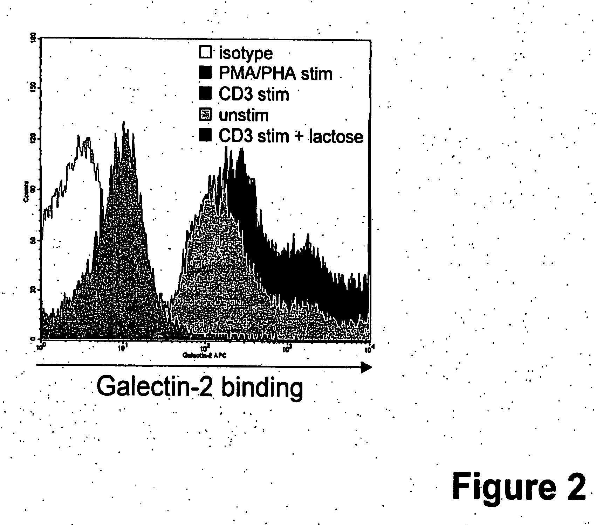

**Figure 2 shows β-galactoside dependent binding of galectin-2 to T cells.** Cells were cultured in the presence of 5 µg/ml galectin-2 coupled to biotin and various combinations of cross-linked anti-CD3 mAb, PMA/PHA, or lactose (50 mMol) for 24h. Cells were then harvested and binding of galectin-2 to T cells determined by two-color flow cytometry using streptavidine labelled-APC to detect biotinylated sites. The graph is representative for three to five different experiments.

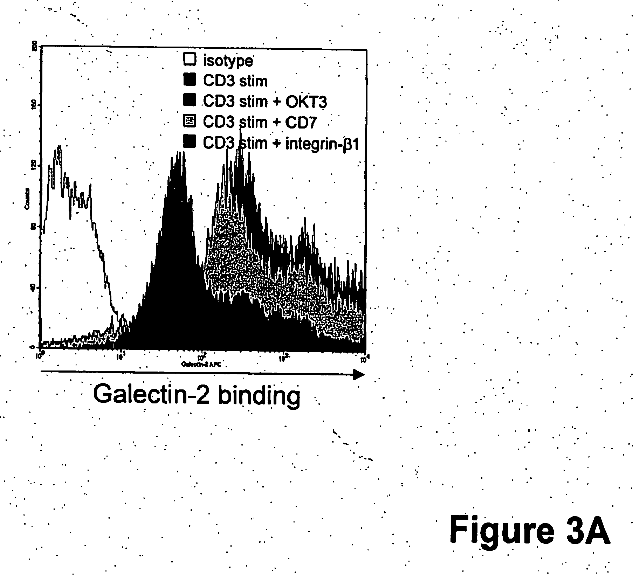

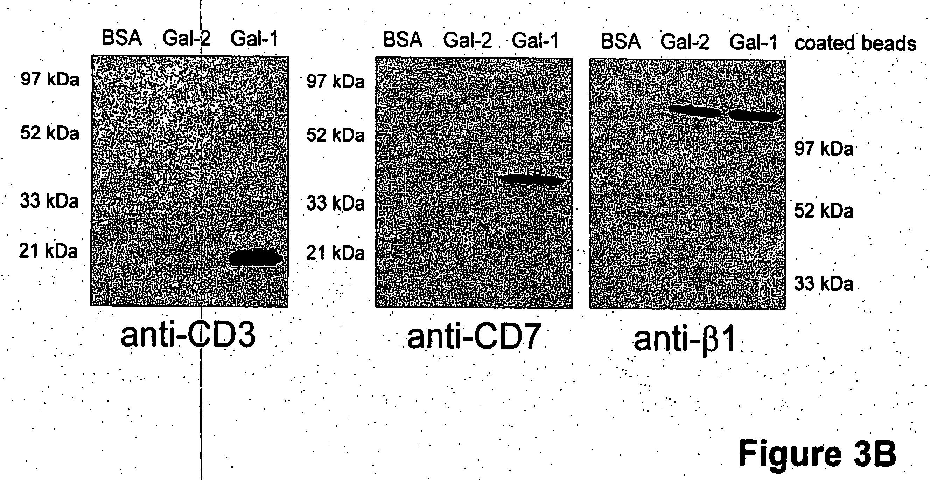

**Figure 3 shows that galectin-2 binds to β1 integrin, but not CD3 or CD7.** A) Cells were preincubated with CD3, CD7, and β1 mAb (1:100 dilution) for 1h at RT and then cultured for additional 24h in the presence of 5 µg/ml galectin-2 coupled to biotin. Cells were then harvested and binding of galectin-2 to T cells determined by two-color flow cytometry using streptavidin labelled-APC to detect biotinylated sites. B) Galectin-1 coimmunoprecipitates with CD3, CD7 and β1 integrin, whereas galectin-2 coimmunoprecipitate only with integrin β1. Cells were incubated with galectin-1 or -2 coated tosyl-activated beads and subsequently lysed. Bead-bound cell fragments were separated magnetically and immunoblotted for CD3, CD7 or β1. Both graphs are representative for four different experiments yielding similar results.

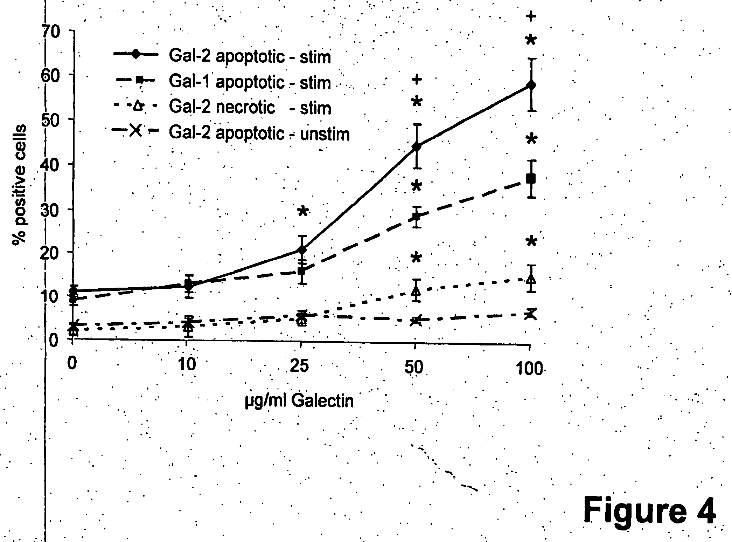

**Figure 4 shows induction of T cell apoptosis by galectin-2**. Analysis of annexin-V and PI (propidium iodide) levels (annexin V binds to phosphatidylserine (PS). PS is flipped during early apoptosis from the inside to the outside of the cell membrane. Annexin-V detects extracellular PS and thus determines the number of apoptotic cells) revealed that galectin-2 induces more T cell apoptosis and necrosis than galectin-1 and that cell activation was required to induce galectin-2 mediated cell death. Cells were cultured with 0, 10, 25, 50, or 100 µg/ml galectin-1 or -2 in the presence or absence of cross-linked anti-CD3 mAb for 24h. Apoptosis and necrosis were assessed by annexin-V and PI staining, respectively. Data represent mean±SEM of six to seven independent experiments. *p<0.05 for increase vs. 0 µg/ml galectin; **p<0.05 for galectin-2 vs. galectin-1.

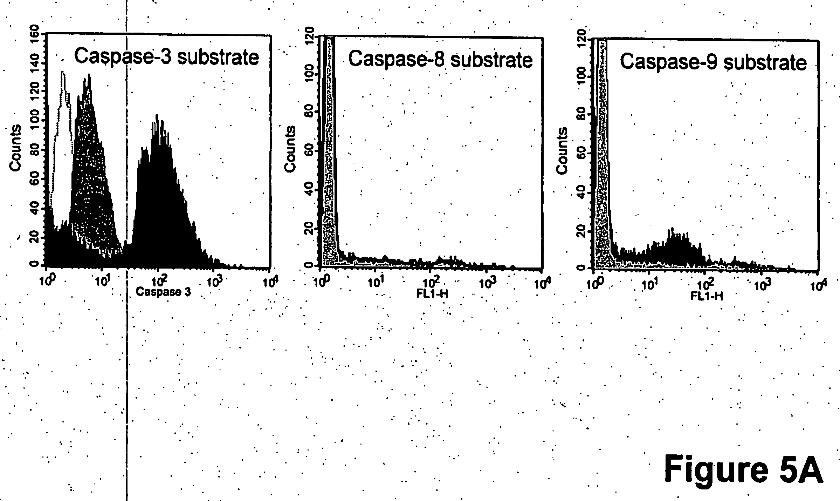

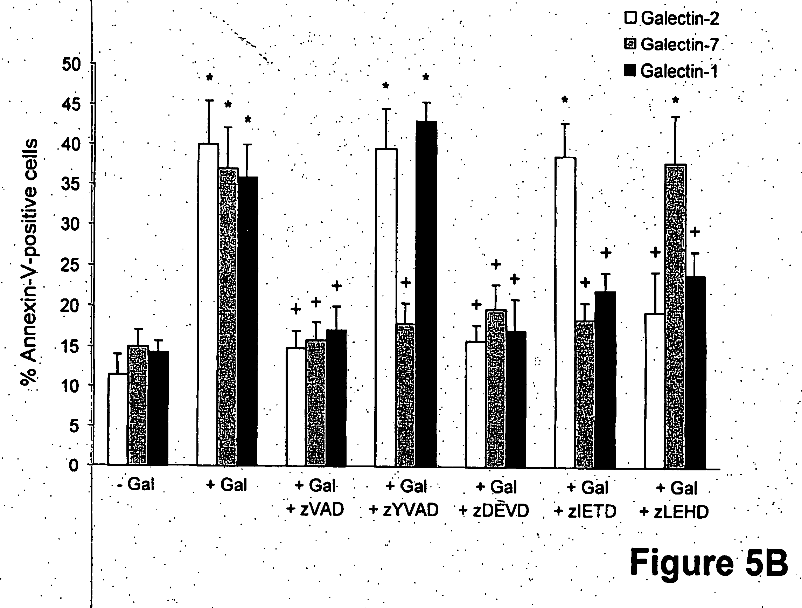

**Figure 5 shows that galectin-2 mediated apoptosis is caspase-3 and 9 dependent.** A) Gal-2 induces activity of caspases-3 and -9, but not of caspase-8 (A). Cells were incubated in the presence or absence of 50 µg/ml Gal-2 for 24 h and caspase activity was assessed by flow cytofluorometry as described in Material and Methods. Illustrated plots are representative for four independent experiments which yielded very comparable results. B) The pan-caspase inhibitor zVAD and the caspase-3 inhibitor (zDEVD) impaired the activity of galectins-2, -7 and -1 to induce cell death. The caspase-1 inhibitor zYVAD blocked only Gal-7 induced cell death, whereas the caspase-9 inhibitor zLEHD blocked Gal-2- and -1- induced apoptosis. The caspase-8 inhibitor zIETD blocked Gal-7- and -1- induced cell death (B). Data represent mean ± SEM of four independent series of experiments. *p < 0.05 for increase vs. no presence of galectin; ⁺p < 0.05 for decrease vs. galectin.

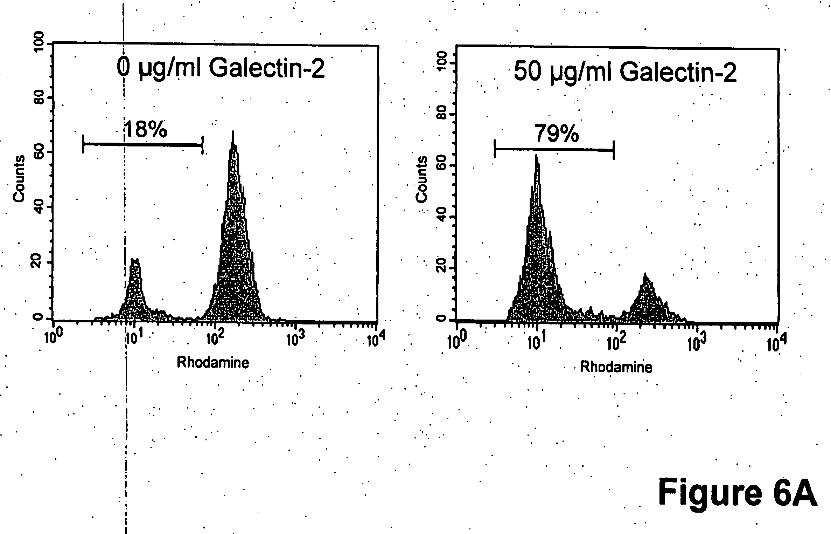

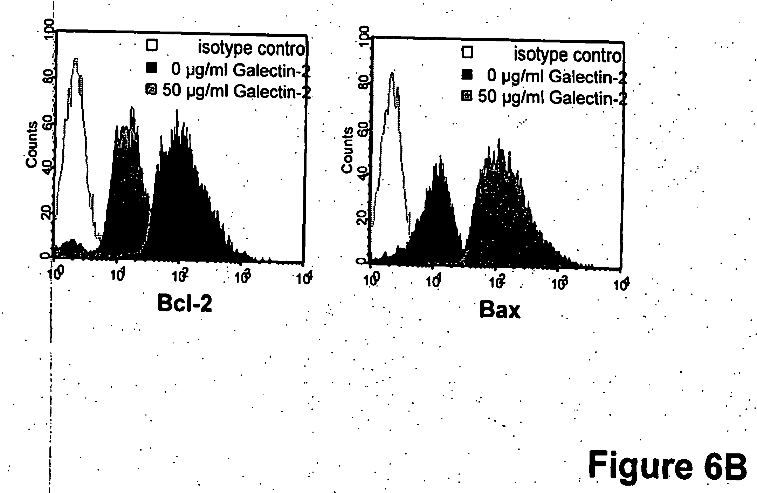

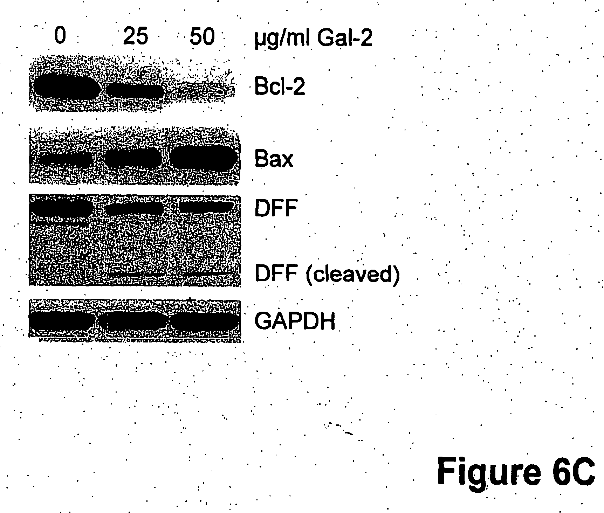

**Figure 6 shows that galectin-2 disrupts the mitochondrial membrane potential,** decreases **bcl-2, but increases bax protein levels and induces cleavage of the DNA-fragmentation factor. Cells were cultured for 48h in the presence of 0, 25 or 50 µg/ml galectin-2.** A) Rhodamine 123 staining demonstrates a reduction of the mitochondrial membrane potential by galectin-2. B) Flow cytometry and C) western blotting show that galectin-2 profoundly reduces anti-apoptotic bcl-2, while increasing pro-apoptotic bax levels and inducing DFF-cleavage. All panels are representative of three different experiments.

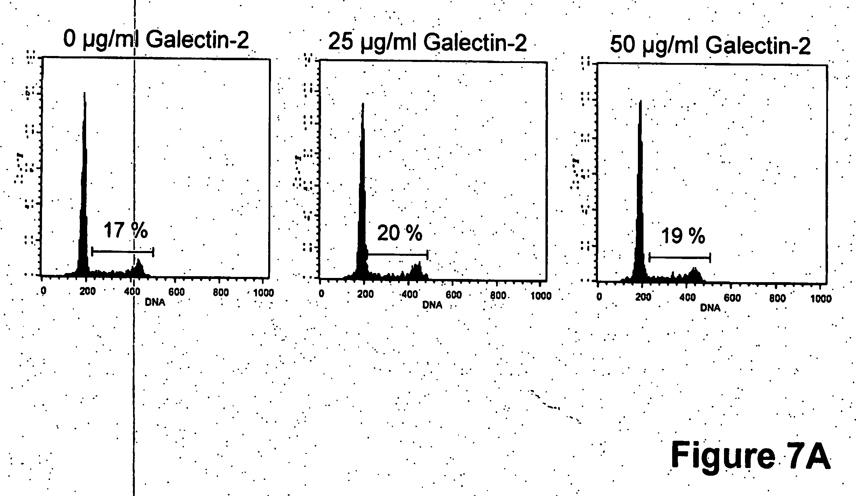

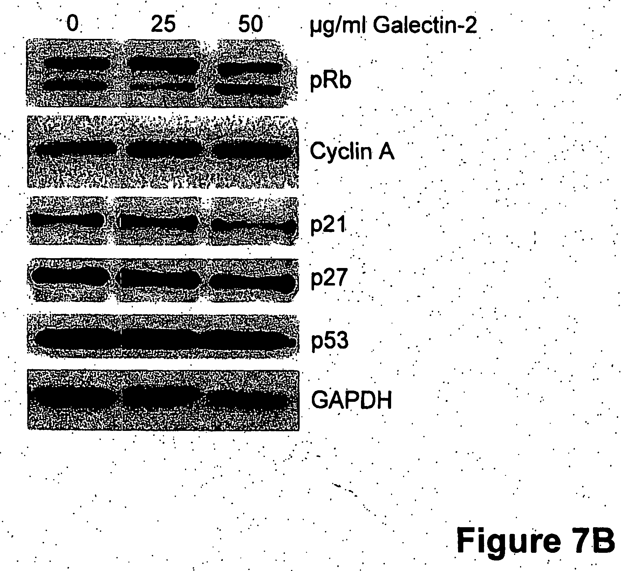

**Figure 7 shows that galectin-2 does not alter T cell cycling.** A) The number of cycling cells in S-, or G2/M-phase is comparable in the presence or absence of galectin-2. Cells were cultured with 0, 25 or 50 µg/ml galectin-2 and activated with cross-linked anti-CD3 monoclonal antibody for 72h. Cell cycle phases were assessed by measuring DNA content by PI staining followed by flow cytometry. Each panel is representative of five different experiments. B) Cell cycle regulator expression is unchanged by galectin-2. Cells were cultured with 0, 25 or 50 µg/ml galectin-2 and activated with cross-linked anti-CD3 mAb for 72h after which protein expression was assessed by western blotting. Each panel is representative of at least four different experiments.

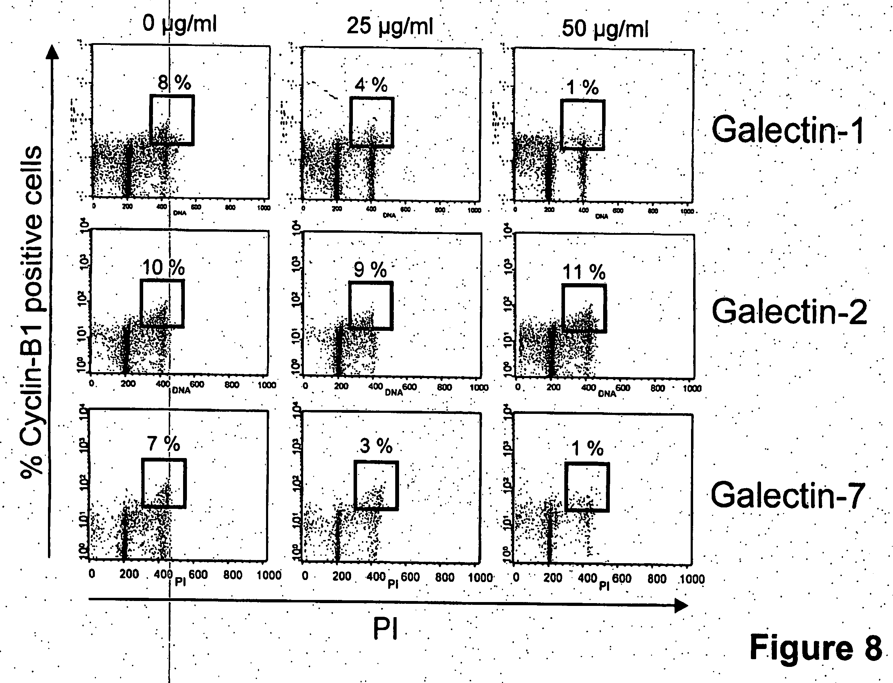

**Figure 8 shows that galectin-1 and -7, but not 2 inhibit G2/M-cell cycle phase progression**. Compared to galectin-2, flow cytometric analysis revealed that galectin-1 and -7decrease cyclin B1 expression in the G2/M phase of activated PBT. Cells were cultured in the presence of cross-linked CD3 mAb for 72h, after which cyclin B 1 expression and DNA content were examined by flow cytometry. The fig. is representative for four different experiments.

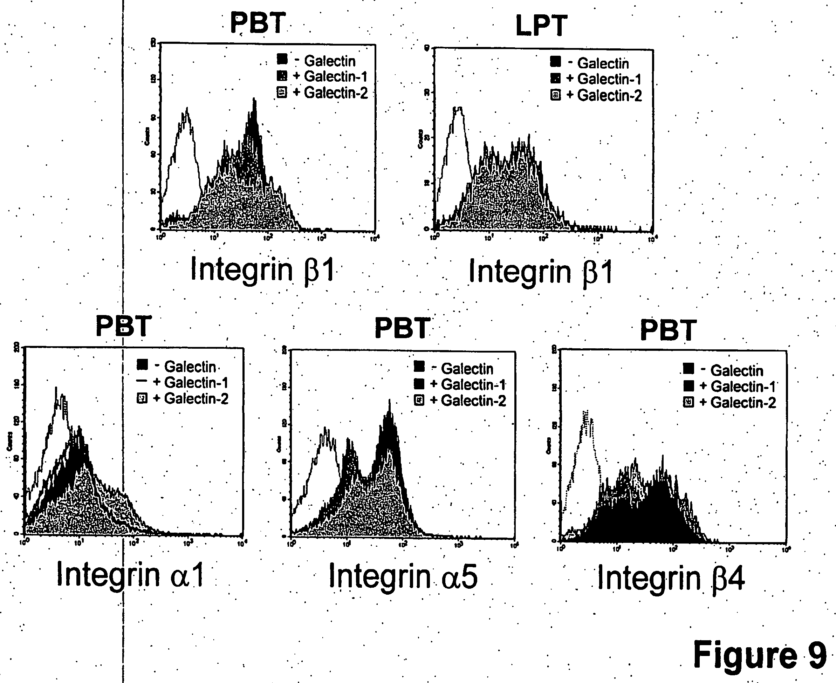

**Figure 9 shows that galectin-2 distinctively alters integrin expression.** In PBT, galectin-1 and -2 downregulates integrin β1 and α5 comparably, does not change β4, and slightly increase α1 expression. In LPT, galectin-1 and -2 fails to modulate integrin β1 expression. The dotted line indicates the isotype. Cells were incubated in the presence or absence of 50 µg/ml galectin-1 or -2 and integrin expression was assessed by flow cytometry. Negative control cells were gated to contain less than 3% positive cells. Representative histograms of three experiments are shown.

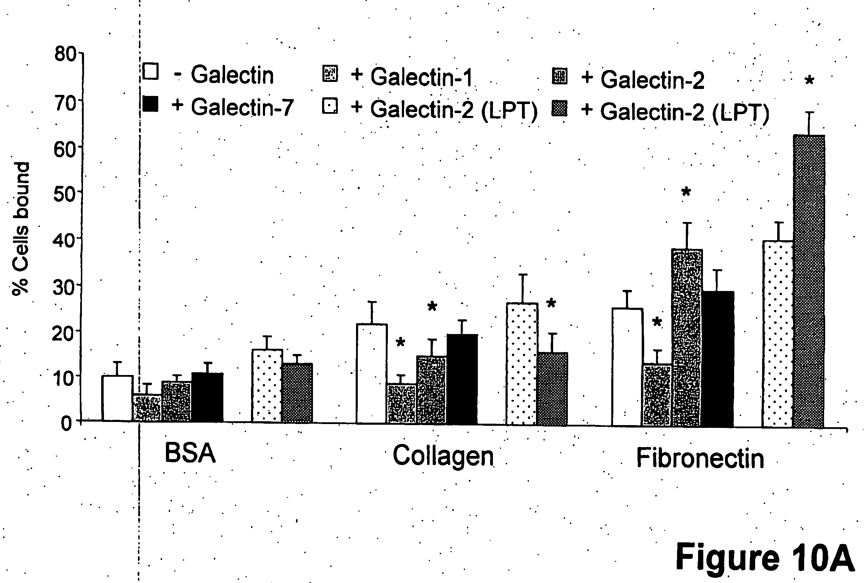

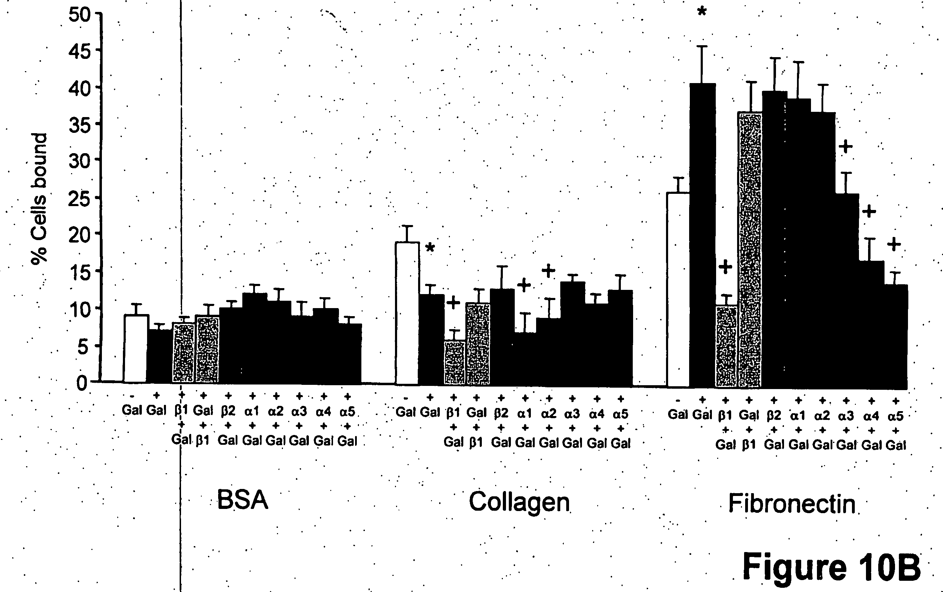

**Figure 10 shows that galectin-2 distinctively modulates T cell adhesion to collagen I and fibronectin.** A) Galectin-1 inhibits cell adhesion to collagen type I and fibronectin, while galectin-2 inhibits PBT and LPT adhesion to collagen and increases T cell attachment to fibronectin. Galectin-7 does not significantly alter T cell adhesion to collagen or fibronectin. B) Specific blocking integrin mAb inhibit galectin-2 mediated modulation of cell adhesion to collagen type I and fibronectin. Pre-incubation of T cells with galectin-2 before adding integrin-β1 mAb reversed the inhibiting effect of the integrin β1 mAb. PBT and LPT were labelled with calcein and 5x10⁵ cells/well were allowed to adhere for 2 hrs to BSA-coated plastic as a control, collagen type 1, or fibronectin. Non-adherent cells were removed by washing. The fluorescence of adherent T cells was quantified using a fluorescence spectrophotometer. Data represent mean±SEM of five independent experiments. *p<0.05 vs 0 µg/ml Gal-2; ⁺p<0.05 vs 25 µg/ml Gal-2.

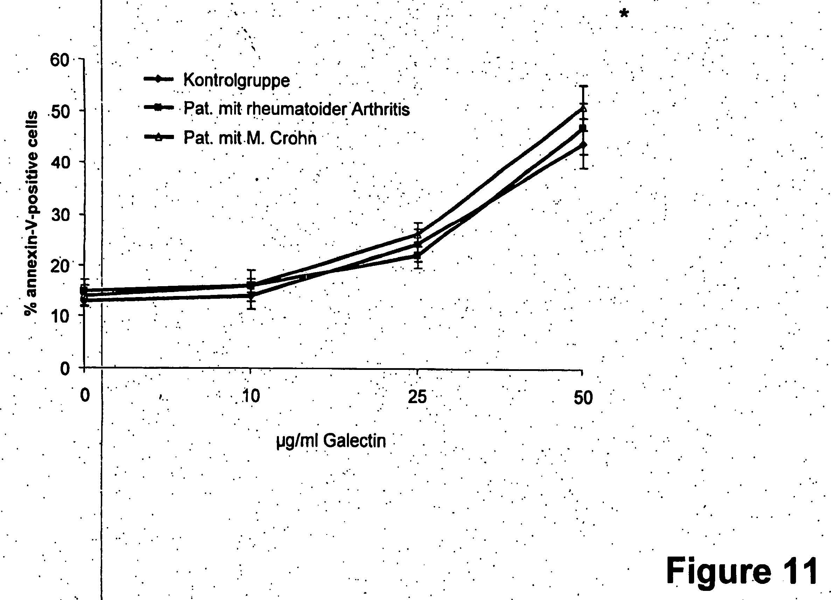

**Figure 11 shows the effect of varying concentrations of galectin-2 on T-cells from** pa**tients having RA or Crohn's disease and a control group.** In detail, PBT from patients with CD and RA and healthy controls were stimulated with cross-linked anti-CD3 mAb antibodies for 48 hours in the presence and absence of the indicated concentration of human galectin-2. Apoptosis was determined by annexin-V staining.

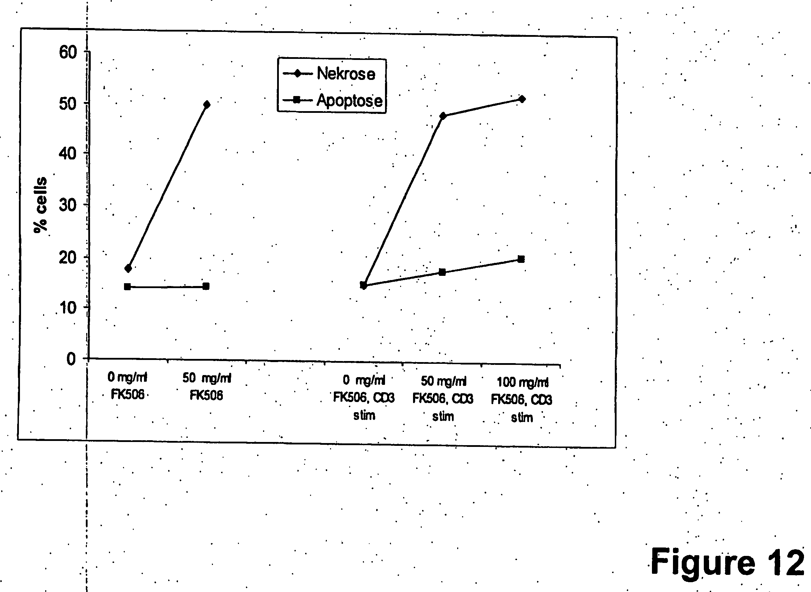

**Figure 12 shows the effect of tacrolimus (Prograf®) on the apoptosis and necrosis of stimulated and unstimulated PBT.** Tacrolimus is one of the most potent immunosuppressants and is currently being used for the prevention of organ rejection following solid organ transplantation and the treatment of inflammatory bowel diseases in selected patients. Yet it mainly induces a necrosis and no apoptosis of unstimulated as well as anti-CD3 stimulated PBT. In contrast thereto and as shown in figure 4 galectin-2 makes this distinction and it mainly induces apoptosis in PBT, thus indicating a better suitability of galectin-2 for the treatment of inflammatory bowel diseases.

### Examples

### Example 1: Materials and methods

### Reagents and antibodies

CD3 mAb (mAb)(OKT3; Ortho Diagnostic System Inc., Raritan, NJ), CD2 mAb (T11₂/T11₃; generously provided by Dr. Ellis Reinherz, Boston, MA), PMA (Sigma-Aldrich, St. Louis, MO) and PHA (Gibco, Grand Island, NY) were used for T cell activation. The broad spectrum caspase inhibitor Z-Val-Ala-Asp(OMe)-fluoromethylketone (zVAD-fmk) was purchased from Biomol (Plymouth Meeting, PA), and caspase-1 inhibitor Z-Tyr-Val-Ala-Asp(Ome)-Ch₂F (Z-YVAD-fmk), caspase-2 inhibitor Z-Val-Asp-Val-Ala-Asp(OMe)-fluoromethylketone (zVDVAD-fmk), caspase-3 inhibitor Z-Asp-Glu-Val-Asp(OMe)-fluoromethylketone (zDEVD-fmk), caspase-8 inhibitor Z-Ile-Glu-Thr-Asp(OMe)-fluoromethylketone (zIETD-fmk) and caspase-9 inhibitor Z-Leu=Glu(Ome)-His-Asp-(Ome)-Ch₂F (Z-LEHD-fmk) were purchased from Calbiochem (San Diego, CA). FITC-conjugated anti-cyclin B1 and PE-labelled anti-active caspase 3 were purchased from BD Pharmingen (San Diego, CA), CD3-PE, CD4-PE, CD8-FITC, CD3-PE, FITC- and PE-labelled polyclonal anti-mouse IgG were obtained from DAKO (Dako, Carpenteria, CA). Secondary FITC-labelled goat-anti mouse was purchased from Biosource (Camarillo, CA) and APC-labelled streptavidine was obtained from Caltag (Burlingame, CA). The Carboxyfluorescein (FAM) caspase detection kits, measuring caspase activity, were obtained from Biocarta (San Diego, CA). Lactose, sucrose, cyclophosphamide, and rhodamine 123 were purchased from Sigma-Aldrich. Propidium iodide (PI) was purchased from Calbiochem (San Diego, CA). All protease- and phosphatase inhibitors used for western blotting were purchased from Sigma-Aldrich. The antibodies against human caspase 3, DFF, PARP, retinoblastoma (Rb) protein, cyclin A, p21, p27, and p53 were purchased from BD Pharmingen. Antibodies against human bax, bcl-2, and cytochrome c were obtained from Santa Cruz Biotechnologies (Santa Cruz, CA). The cytometric bead array kit was purchased from BD Pharmingen, and IFN-γ, IL-10, and IL-2 ELISA kits were obtained from R&D (Minneapolis, MN). Galectin-2 and -7, as well as Galectin primers were kindly provided by H.-J. Gabius (Institute of Physiological Chemistry, Faculty of Veterinary Medicine, Munich, Germany). For flow cytometry and blocking experiments, anti-human α1 integrin mAb (clone FB12), anti-human integrin α2 mAb (clone P1E6), anti-human integrin α3 mAb (clone P1B5), anti-human integrin α4 (clone P1H4), anti-human integrin α5 (clone P1D6), anti-human integrin β2 (clone P4C10) and β2 (clone 3E1) were used (all from Chemicon, Temecula, CA).

### Preparation of T lymphocytes

PBMC (peripheral blood mononuclear cells) from healthy volunteers were isolated from heparinized venous blood using Ficoll-Hypaque density gradients. For isolation of peripheral blood T lymphocytes (PBT), PBMC cells were incubated for 30 minutes at 4°C with magnetically-labelled CD19, CD14, and CD16 Ab directed against B-lymphocytes, monocytes, and neutrophils, respectively (Miltenyi Biotec Inc., Bergisch-Gladbach, Germany). T cells were then collected using a magnetic cell sorting system (MACS, Miltenyi Biotec Inc.). Lamina propria T cells (LPT) were isolated from surgical specimen obtained from patients admitted for bowel resection for malignant and non-malignant conditions of the large bowel, including colon cancer and benign polyps as previously described (21). Briefly, the dissected intestinal mucosa was freed of mucus and epithelial cells in sequential washing steps with DTT and EDTA, and digested overnight at 37°C with collagenase and DNase. Mononuclear cells were separated from the crude cell suspension by layering on a Ficoll-Hypaque density gradient. For LPT purification, macrophage-depleted lamina propria mononuclear cells were incubated for 30 minutes at 4°C with magnetically-labelled beads as described above and collected by negative selection using the MACS system. As assessed by flow cytometry, the purified PBT and LPT populations contained >99% and >92% CD3⁺ cells, respectively.

### PCR

Total RNA was isolated using the Advantage RT-for-PCR-kit (Clontech, Palo Alto, CA) according to the manufactor's instruction. Two point five µg aliquots of total RNA were reverse transcribed essentially as previously described (8) and Galectin-2 mRNA amplified using the following primers: sense, 5'-ATGACGGGGGAACTTGAGGTT-3' and anti-sense 5'-TTACGCTCAGGTAGCTCAGGT-3'. The thermal cycle commenced with a hot start 94°C for 4 minutes followed by 36 cycles each consisting of 94°C for 60 seconds, annealing for 60 seconds at 60°C, extension at 72°C for 120 seconds, and terminated after a final 10 minute period at 72°C. The products were separated on a 1% TAE agarose gel and visualized by ethidium bromide staining under UV light.

### Western blotting

For immunobloting cells were washed in PBS, and lysed in cell lysis buffer (1% Triton-X, 0.5% NP-40, 0.1% SDS, 0.5% sodium deoxycholate, 5mM EDTA, 50 mM and 50 mM protease- and phosphatase-inhibitor cocktail 2, 1 mM PMSF, 100 µg/ml trypsin-chymotrypsin inhibitor, 100 µg/ml chymostatin in PBS). The concentration of proteins in each lysate was measured using the Bio-Rad protein assay (Bio-Rad Laboratories, Hercules, CA). Equivalent amounts of protein (10 µg) were fractionated on a 10-20% Tris-glycine gel and electrotransferred to a nitrocellulose membrane (Novex, San Diego, CA). Membranes were blocked with 5% milk in 0.1% Tween 20-PBS (Fisher Scientific, Hanover Park, IL), followed by incubation for 60 min at room temperature with the indicated primary antibody. The membranes were washed six times with 0.1% Tween 20-PBS and then incubated for 1 h with the appropriate horseradish peroxidase-conjugated secondary antibody (Santa Cruz Biotechnology), washed, and incubated with the chemiluminescent substrate (Perkin-Elmer, Carlsbad, CA) for 5 minutes. The membranes were then exposed to film (Amersham, Arlington Heights, IL).

### Determination of galectin-2 binding

To measure galectin-2 binding to T-lymphocytes, cells were stimulated for 1, 6, 12, 24, 48, and 72h with or without anti-CD3 mAb or PMA and PHA and incubated in the presence of 5 µg/ml biotin-labelled galectin-2 or galecin-7. Cells were then harvested, washed and stained with anti-CD3-FITC mAb and APC-conjugated streptavidine (Biosource) followed by flow cytometric analysis. Each analysis was performed on at least 20.000 events.

### Magnetic separation of galectin-2 containing complexes

For separation of galectin-2-associated complexes, 1x10⁸ tosylactivated, supermagnetic, polysterene coated beads (Dynalbeads, Dynal Biotech, Oslo, Norway) were coated either with 350 µg BSA, Galectin-1, Galectin-2, CD7, integrin-β1 or OKT3 overnight at 37°C using tilt rotation. After incubation, the beads were washed in 0.2 M Tris (pH 8.5 containing 0.1% BSA), and then incubated with 2x10⁶ PBT for 1 h at 37°C. Unconjugated cells were removed by extensive washing and cells were subsequently lysed with homogenization buffer (20 mM Tris HCl, pH 7.6; 10 mM MgCl₂, 0.05% Triton-X 100, 50 mM phosphatase- and 50 mM protease inhibitor cocktail), resuspended in SDS-DTT protein loading buffer, heated to 95°C for 5 min and submitted to SDS-PAGE electrophoresis.

### Adhesion assay

96 well flat-bottom plates were precoated overnight at 4°C with 12 µg/well fibronectin (Chemicon) in DPBS, 40 µg/well collagen type I (Sigma-Aldrich) in 0.1 M acetic acid, or 3% BSA (Sigma-Aldrich) in DPBS as a control and then washed three times with DPBS. Freshly isolated T cells were fluorescently-labelled with 5 µM calcein-AM (Molecular Probes, Eugene, OR) for 30 min at 37°C in 5% CO₂ at 5 x 10⁶ cells/ml in RPMI. The cells were then washed three times with RPMI containing 5% FCS. Calcein-labelled T cells were resuspended in RPMI and 5 x 10⁵ T cells/well were added to 96-well plates containing purified ECM components or BSA for the indicated times. Saturating concentrations of integrin-blocking mAbs (predetermined by flow cytometry) were preincubated with T cells for 30 min at 37°C before adding T cells to the wells for the adhesion assay. Nonadherent cells were removed by a standardized washing technique that was developed to minimize background binding, which includes both an orbital and a rocking motion repeated three times with RPMI. Adhesion was quantified with a multiwell fluorescent spectrophotometer (Tecan, Groedig, Austria). For each experimental group the results were expressed as the mean percentage ± SD of bound T cells from triplicate wells.

### Determination of apoptosis and cell death

Apoptosis was determined by monitoring nuclear fragmentation and externalization of phosphatidylserine (PS). To detect nuclear fragmentation, T cells were cultured in the presence and absence of the respective galectins, with or without blocking antibodies or further activation, for 48h. Cells were then fixed in 0.2 ml of 37% formaldehyde for 10 min at room temperature. The cells were then treated with 1 ml PBS containing 0.2% Nonidet P-40 (Sigma-Aldrich) for 2 min at room temperature and then rinsed once in PBS. The apoptotic cells were distinguished by staining of nuclear DNA with 4,6-diamino-2-phenylindole (DAPI) (Calbiochem). DAPI was added at a concentration of 0.2 ng/ml of PBS and incubated at room temperature for 20 min. Cells were rinsed twice in PBS and the coverslips were maintained cell side down on a microscope slide and analysed using a Zeiss Axiovert 135M microscope (Carl Zeiss, Oberkochen, Germany). To analyse early phases of apoptosis, externalization of PS was measured. Cells were cultured as described, harvested at the respective timepoints, stained with FITC-labelled annexin-V and PI and analysed by flow cytometry using the CellQuest software program (BD Pharmingen). A minimum of 15,000 cells was analysed in each case.

### Assessment of mitochondrial membrane potential

Rhodamine 123 is a fluorescent cationic dye that accumulates in the mitochondrial matrix because of its charge and solubility in both the inner mitochondrial membrane and matrix space (22). Based on the observation that accumulation of this lipophilic dye is in proportion to ΔΨ, and that deenergizing of the mitochondria decreases rhodamine 123 fluorescence (23), the inventors measured the mitochondrial membrane potential of PBT. Cells were stimulated in the presence or absence of galectin-2 with CD3 for 24h, harvested, washed and resuspended in 1ml of rhodamine 123 (10µg/ml, Sigma-Aldrich) for 30 min at 37°C in the dark. The samples were washed twice in cold PBS and fluorescence analysis by flow cytometry using an argon ion laser with an emission filter at 530nm was performed immediately without fixation (BD FacsCalibur). PBT without stimulation and unstained samples were used as controls.

### Flow cytometric analyses

For surface staining, cells were washed twice in ice-cold PBS and 1x10⁶ cells were resuspended in flow buffer (HBSS containing 1% BSA and 0.1% sodium azide). Cells were incubated with the respective mAb at predetermined saturating concentrations or with isotype-matched nonspecific mouse mAb (DAKO) for 30 min at 4°C, washed twice with flow buffer, and incubated with FITC-conjugated goat anti-mouse IgG for 30 min at 4°C. The cells were again washed twice with flow buffer, fixed in 1% paraformaldehyde and analyzed by a single laser flow cytometer (FacsCalibur, Beckman Coulter), using Cell Quest software (BD Pharmingen). Cells stained with a negative control Ab were gated to contain <2% positive cells. To perform analysis of intracellular proteins, cells were washed twice with PBS, adjusted to 1 x 10⁶ cells per sample, and fixed in 90% methanol at -20°C. After fixation, cells were washed twice with PBS and incubated with a monoclonal mouse anti-bax, Bcl-2, Rb, Cyclin A, p21, p27, or p53 antibody for 45 minutes at 4°C, followed by an incubation with a goat anti-mouse FITC-conjugated mAb (Biosource) for 45 minutes at 4°C. Thereafter cells were washed and analyzed by flow cytometry as described above. The background level of immunofluorescence was determined by incubating cells with FITC-conjugated mouse IgG.

### Determination of caspase activity

To measure caspase activity, 5x10⁵ PBT were incubated in the presence or absence of 50 µg/ml galectin-2 together with derivates of the PE-labelled affinity purified anti-caspase-3 Ab (BD Pharmingen), the carboxyfluorescein-labelled caspase-8 inhibitor FAM-LETD-fmk or caspase-9 inhibitor FAM-LEHD-fmk (both from Biocharta, Hamburg, Germany), all binding irreversibly to activated caspase-3, -8 or -9, respectively. Cells were than analyzed by flow cytometry (FACS-Calibur, BD) and the increase of caspase activity determined after proper gating in correlation to untreated cells.

### Analysis of T cell cycling

Flow cytometry was performed after staining for DNA content, and cyclin B1, essentially as previously described (21). Briefly, cells were washed twice with PBS, adjusted to 1x10⁶ cells/sample and fixed in 90% Methanol at -20°C. After fixation, cells were washed twice with PBS, incubated for 45 min at 4°C with a cyclin B1-FITC conjugated monoclonal antibody. After the final wash, cells were resupended in PBS and 5 µl of RNase (0.6 pg/ml, 30-60 Kunitz Units, Sigma), incubated at 37°C for 15 min and the chilled on ice. One hundred and twenty five microliters of PI (200µg/ml) were added prior to analysis by flow cytometry. Each analysis was performed on at least 25.000 events.

### Statistical analysis

Statistical analysis was performed using the paired student's t test. Results are expressed as mean±SEM, and significance was inferred with p values<0.05.

### Example 2: Results

### Expression of galectin-2 in T cells

Whereas galectin-1 and -3 are widely distributed among various mammalian tissues and cell types, other galectins such as galectin-4 and -6 are expressed in a more tissue restricted fashion (6-8). The expression of galectin-2 is different from other galectins and seems to be restricted to the GI tract (8, 9). Within the GI tract, galectin-2 mRNA expression was detected in the duodenum, jejunum, and to a lesser extent in the coecum, colon and rectum (9), however protein expression in human tissues has not yet been examined. Since galectins exert strong immunoregulatory effects, the inventors first wanted to know, if galectin-2 is expressed in resting and activated peripheral and intestinal T cells. PCR analysis demonstrated, that in contrast to galectin-1, galectin-2 mRNA is not expressed in resting or activated PBT (Fig. 1a). Consequently, western blot analysis failed to detect protein expression of galectin-2 in resting or stimulated PBT or LPT (Fig. 1b). As positive control the rat non-transformed intestinal epithelial cell line IEC-6 was used, demonstrating galectin-2 expression in these cell lines (Fig. 1b).

### β-galactoside dependent binding of galectin-2 to T cells

To examine if galectin-2 exerts its immuno-regulatory function by binding to T cells, galectin-2 was labelled with biotin and added to non-activated, anti-CD3, or PMA and PHA activated PBT for increasing periods of time. Flow cytometric analysis showed that half-maximum binding of galectin-2 occurred in all three groups within 60 min of incubation, reached a maximum at 6h and remained bound over the next three days (Fig. 2). The binding of galectin-2 to PBT was inhibited by the β-galactoside lactose, but not sucrose (Fig. 2).

### Galectin-2 binds to β1 integrin

In order to study the interaction between galectin-2 and T cells further, the present inventors screened several mAbs which have been shown to block galectin-1 binding to transformed human T cell lines (24). The inventors therefore pre-incubated freshly isolated PBT with Abs recognizing CD3, CD7, and integrin β1 for 1h and added then biotinylated galectin-2 for additional 24h. When galectin-2 binding to T cells was determined by flow cytometry, pre-incubation with the blocking integrin β1 mAb reduced binding of galectin-2 to T cells 48.2 ± 9.4 %, whereas in contrast to galectin-1 (24), CD3 and CD7 failed to inhibit galectin-2 binding to T cells. (Fig. 3a).

To isolate galectin-2 containing complexes directly, the inventors performed immunoprecipitation using tosyl-activated magnetic beads. Beads were coated with galectin-1, galectin-2, or BSA for 24h and then incubated with purified T cells. After immunoprecipitation, cell lysates were immunoblotted against CD3, CD7 and integrin β1. As shown in fig. 3b, galectin-1 binds to CD3, CD7, and β1, whereas galectin-2 binds only to β1, but not CD3 or CD7.

### Induction of T cell apoptosis by galectin-2

Galectins have a variety of modulatory effects in T cells and can induce, but also inhibit T cell apoptosis (25, 26). Since it is not known if galectin-2 modulates T cell apoptosis, the inventors first assessed if galectin-2 induces surface exposure of phospatidylserine, as measured by annexin-V staining, in activated and non-activated PBT. When PBT were cultured in the presence of 0, 10, 25, 50 and 100 µg/ml galectin-2, but without cell stimulation, galectin-2 failed to induce T cells apoptosis (Fig. 4). In contrast, when PBT were activated by anti-CD3 mAbs, galectin-2 induced a dose-dependent apoptosis, but also, albeit to a lesser extent, necrosis, of T cells (Fig. 4). In contrast thereto, the immunosuppressent tacrolimus (FK506), which is commonly used to prevent organ rejection following allogenic transplantation of kidneys and livers and to treat refractory inflammatory bowel diseases in selected patients, makes no distinction between stimulated and unstimulated T-cells and mainly induces necrosis (Fig. 12) thus causing possibly septic effects. The percentage of T cell apoptosis induced by galectin-2 was higher than that induced by galectin-1 (Fig. 4), and was comparable in CD4 and CD8 positive T cell populations (data not shown). To examine the requirement of de-novo protein synthesis for galectin-2 induced cell death, cells were exposed to 0, 10, and 1000 nM of cyclophosphamide, a protein synthesis inhibitor (see Kashio, Y., K. Nakamura, M. J. Abedin, M. Seki, N. Nishi, N. Yoshida, T. Nakamura, and M. Hirashima. 2003. Galectin-9 induces apoptosis through the calcium-calpain-caspase-1 pathway. *J.Immunol.* 170:3631), in the presence and absence of galectin-2. Furthermore, the inventors examined the effect of lactose on the ability of galectin-2 to induce apoptosis. Whereas cyclophosphamide failed to modulate galectin-2 induced T cell death (data not shown), apoptosis was largely inhibited by 50 mM lactose, but not sucrose.

### Galectin-2 mediated apoptosis is caspase-3 and -9 dependent

Having established that galectin-2 induces T cell apoptosis, the inventors were interested to assess the apoptotic pathways used by galectin-2. They therefore measured caspase activities in galectin-2 treated, anti-CD3 activated PBT using fluorogenic substrate assays. When PBT were activated by the CD3 pathway, galectin-2 induced a significant increase in caspase-3 and -9, but not -8 activity (Fig. 5a). To assess the functional relevance of this finding, the inventors evaluated the effect of caspase inhibition on galectin-2 induced T cell apoptosis. Both specific and broad spectrum caspase inhibitors were tested, and all contained fluoromethylketone (fmk), which renders the inhibitory effect irreversible (all used at 50 µM). Caspase-1 (zYVAD) and -8 (zIETD) inhibition had no effect on galectin-2 induced T cell apoptosis. In contrast, caspase-3 (zDEVD), -9 (zLEHD) and the broad spectrum caspase inhibitor (zVAD) significantly inhibited galectin-2 induced cell death (Fig. 5b). Since it has been shown that monomeric galectin-1 increases both, caspase-8 and -9 activity (27), and the tandem-repeattype galectin-9 induces apoptosis via caspase-1, but not -8 or -9 (28), the inventors also assessed the effect of galectin-7, like galectin-2 a monomeric prototype galectin (2), on T cell apoptosis. Although galectin-7 induced PBT apoptosis comparable to galectin-2, preincubation with zVAD, zDEVD, and zIETD, but not zLEHD inhibited galectin-7 mediated cell death, indicating that galectin-7 mediates T cell apoptosis via caspase-3 and -8, but not-9 (Fig. 5b). Having documented the pattern of caspase activation by Gal-2, the inventors proceeded to include Gal-1 in aliquots of our cell preparations comparatively. The ability of zVAD to reduce Gal-1-induced cell death provided evidence that caspases are involved in Gal-1-mediated apoptosis (Fig. 5B). A conspicuous difference was the marked effect of the caspase-8 inhibitor (Fig. 5B). Gal-1-induced apoptosis thus has ist own profile with involvement of caspases-3, -8 and -9. Assays with Gal-7, another homodimeric proto-type galectin, similarly revealed pro-apoptotic activity of the stimulated T cells. Inhibition of caspases-1, -3 and -8, but not of caspase-9 reduced its extent (see above). Evidently, caspase involvement is different among the proto-type subgroup of galectins, warranting further study. Here, the present inventors focus on the pathway of inducing apoptosis for Gal-2. With caspase-9 as involved mediator of the determined response to Gal-2 exposure, it appeared likely that Gal-2 makes use of the intrinsic pathway, a suggestion testable by looking at the mitochondrial membrane potential. Thus, the inventors next proceeded to analyze this parameter exploiting the redistribution of rhodamine 123.

**Galectin-2-mediated apoptosis is dependent on caspase-3 and -9.** Galectin-2 induces caspase-3 and -9, but not -8, activity (A). Cells were incubated in the presence or absence of 50 µg/ml galectin-2 for 24h and caspase activity was assessed by flow cytometry as described in Material and Methods. Illustrated plots are representative for four independent experiments which yielded comparable results. The pan-caspase inhibitor zVAD and the caspase-3 inhibitor (zDEVD) impair the activity of galectins-2, -7 and -1 to induce cell death. The caspase-1 inhibitor zYVAD blocked only gal-7 induced cell death, whereas the caspase-9 inhibitor zLEHD blocked gal-2 and -1 induced apoptosis. The caspase-8 inhibitor zIETD blocked galectin-7 and -1 induced cell death (B). Data represent mean±SEM of four independent series of experiments. *p<0.05 for increase vs. no presence of galectin; ⁺p<0.05 for decrease vs. galectin.

### Galectin-2 modifies the bax/bcl ratio and disrupts the mitochondrial membrane potential

Events critical for apoptosis include the loss of mitochondrial membrane potential, resulting in cytochrome c release and caspase-9 cleavage (29). Having established that galectin-2 induces T cell apoptosis via caspase-9, the inventors next wanted to examine if galectin-2 disrupts the mitochondrial membrane potential, releases cytochrome c and thus uses the "intrinsic" apoptotic pathway. To investigate this possibility, the inventors used the redistribution of the dye rhodamine 123, a lipophilic cation that accumulates in the mitochondrial membrane. When PBT were activated via the CD3 pathway in the presence of 50 µg/ml galectin-2, a clear decrease in the mitochondrial polarization potential compared to cells cultured in the absence of galectin-2 was found (Fig. 6a). Furthermore, galectin-2 dose-dependently increased cytochrome c release in activated, but not resting T cells (data not shown). Apoptosis and survival are also regulated by the relative balance of pro-apoptotic and anti-apoptotic Bcl-2 family members. In addition, since the bax protein forms ion-conducting channels in the lipid bilayers of mitochondria and therefore plays a crucial role in the mitochondrial pathway of apoptosis (30), the inventors investigated if galectin-2 modulates protein expression of the anti-apoptotic bcl-2 and the pro-apoptotic bax proteins in T cells. Immunoblotting and flow cytometry revealed that galectin-2 significantly reduces bcl-2, but increases bax protein expression, resulting in a lower bcl-2/bax ratio (Fig. 6b and c). It has recently been published that galectin-1 induces surface exposure of phosphatidylserine without inducing apoptosis (31), however other groups could not confirm this finding in other cell types (32, 33). To clarify this discrepancy with respect to galectin-2 and PBT, the inventors analyzed downstream pathways of caspase-3 cleavage in T cells. Immunoblotting revealed, that galectin-2 dose-dependently upregulated the DNA-fragmentation factor (DFF)(Fig. 6c), which is proteolytically cleaved by caspase-3 and irreversibly induces DNA fragmentation and thus cell death (34). These data were confirmed by DAPI staining, showing that 25 and 50 µg galectin-2 induce nuclear fragmentation (data not shown) and confirm the execution of apoptosis by galectin-2.

### Galectin-2 does not modulate PBT cell cycling

Resting T cells are protected from antigen induced cell death and thus apoptosis and cell cycle are closely linked with each other (35-37). It is known that galectin-1 inhibits proliferation in murine T cell hybridoma and thymocytes (38). To address the question, if galectin-2 modulates T cell cycling, the inventors performed an analysis of PBT cycling profiles in response to galectin-2, using DNA staining. When gated on the living cell fraction, cell cycle phase distribution was comparable in anti-CD3 activated PBT cultured in the absence or presence of 25, 50, and 100 µg/ml galectin-2 (Fig. 7a). The present inventors next investigated if galectin-2 modulates levels of key regulatory molecules responsible for initiating and advancing each phase of the cell cycle. As determined by western blot analysis, galectin-2 did not alter protein levels of the cell cycle promoters cyclin D2, retinoblastoma protein (Rb), or cyclin A, neither the cell cycle inhibitors 21, p27, and p53 (Fig. 7b). To determine if this effect is distinctive for galectin-2, the inventors also examined the effects of galectin-1, -2, and-7 on cyclin B 1 expression by flow cytometry in conjunction with PI staining to accurately quantify cell cycle progression to the G2/M-phase. When PBT were activated with anti-CD3 mAb and incubated in the presence of galectin-1 or -7, cyclin B1 expression significantly dropped dose-dependently from 8% to 1% and from 7% to 1%, respectively (Fig. 8). In contrast, galectin-2 did not inhibit PBT cell cycle progression, as demonstrated by unchanged cyclin B1 levels (Fig. 8).

### Galectin-2 modulates integrin expression and cell adhesion

Galectins act as modulators of cell adhesion by interacting with the appropriately glycosylated proteins at the cell surface or within the extracellular matrix (ECM)(39). This interaction is mediated by integrins and since the inventors showed above, that galectin-2 bound to integrin β1, the inventors wanted to determine, if galectin-2 alters integrin expression on T cells. When PBT are activated via the CD3 pathway, galectin-1 and -2 downregulated integrin β1 and α5 expression (Fig. 9). In contrast, neither galectin-1 nor galectin-2 changed β4 integrin expression, responsible for T cell homing to the intestinal lamina propria, and slightly upregulated integrin a5 expression, indicating that galectins regulate integrin expression distinctively. Interestingly, when intestinal T cells as source of memory T cells were examined, galectin-2 failed to modulate integrin β1 expression, indicating that the ability of galectin-2 to affect integrin expression is distinct not only for individual integrins, but also for cell differentiation (Fig. 9).

Galectins distinctively modulate T cell adhesion to extracellular matrix and the presence of galectin-1 inhibits T cell adhesion to ECM glycoproteins (40, 41). Having shown that galectin-2 alters integrin expression of T cells, the inventors hypothesized that galectin-2 will also modify T cell adherence to ECM compounds. When PBT were activated via the CD3 pathway, 22.1 ± 3.5% and 26.3 ± 5.6% of the cells adhere to collagen type I or fibronectin, respectively (Fig. 10a). Confirming the known effect of galectin-1 on T cell adhesion (40), galectin-1 substantially decreased T cell attachment to collagen type I (9.2 ± 3.5%) and fibronectin (14.0 ± 4.6%)(Fig. 10a). In contrast, while adhesion to collagen type I was also significantly reduced by galectin-2 (15.1 ± 5.6%; p<0.05), adhesion to fibronectin was significantly enhanced by galectin-2 (39.8 ± 4.9%; p<0.05)(Fig. 10a). Again, the inventors analysed galectin-7 to verify if this feature is specific for galectin-2. As depicted in fig. 10a, galectin-7 failed to significantly alter PBT adhesion to collagen or fibronectin. As shown above, integrin β1 upregulation by CD3 is inhibited by galectin-2 in PBT, but not LPT. However, when the effect of galectin-2 on T cell adhesion was analysed in LPT, comparable to PBT, galectin-2 inhibited cell adhesion to collagen, while increasing attachment to fibronectin (p<0.05; Fig. 10a). To confirm that these effects were mediated by collagen and fibronectin receptors, cells were pre-incubated for 1h with a subset of specific blocking integrin mAbs. After pre-incubation, 25 µg/ml galectin-2 was added and cells were layered on BSA-, collagen- or fibronectin-coated wells. Cell adhesion to collagen was significantly inhibited by β1, α1 and α2 mAbs, whereas the adhesion to fibronectin was blocked by β1, α3, α4, and α5 mAbs (Fig. 10b), indicating that the adhesion of cells to their respective extracellular matrix was mediated by collagen and fibronectin receptors, respectively. As shown above, galectin-2 binds to β1 (Fig. 3a, b). To determine, if this binding may involve β1 integrin on the cell surface and may therefore alter PBT adhesion to ECM, the inventors pre-incubated cells for 1h with galectin-2 and added integrin-β1 mAbs. Cells were then allowed to adhere on the ECM compounds. Interestingly, pre-incubation with galectin-2 before adding integrin-β1 mAb reversed the inhibiting effect of the integrin β1 mAb, when they were added first, indicating that, at least in part, integrin expression sites were neutralized by galectin-2 (Fig. 1 0b).

### Animal model of colitis

Colitis was induced in 8- to 10-week-old, female, Balb/c mice (Jackson Laboratories, Bar Harbor, ME) by adding 5% DSS (Sigma Chemical Co., St. Louis, MO) (Dextran Sodiumsulfate (DSS) to their drinking water and allowing them to drink ad libitum. Groups of mice were treated either with 1 mg/kg BW (body weight) galectin-2 intraperitoneally or PBS 2h before DSS administration. Mice were weighed and inspected for diarrhea and rectal bleeding. Seven days after induction of colitis by DSS, mice were killed, their entire colon was resected, and its length was measured and weighed. The disease activity index (DAI; i.e., the combined score of weight loss and bleeding) was determined according to a standard scoring system.

### The disease activity index:

Loss in body weight: 0 = no loss; 1 = 5% to 10%; 2 = 10% to 15%; 3 = 15% to 20%; 4 ⇒20%.

Hemoccult: 0 = no blood; 2 = positive; 4 = gross blood.

In this experiment the DAI ranges from 0 to 8. The higher the DAI score the more severely the animal had been affected by colitis.

| **Results:** | | | |
|---|---|---|---|
| DAI: Control group: | 6.3 ± 2.30 (mean±SD), | n=24 | |
| Galectin group: | 4.5 ± 2.1, | n=10; | p=0.04 |

The results show that those animals treated with galectin-2 showed a significant reduction of severity of colitis proving that galectin-2 is particularly suitable for the prevention or treatment of inflammatory bowel diseases.

### REFERENCES

1. Gabius, H.-J., S. André, H. Kaltrier, and H. C. Siebert. 2002. The sugar code: functional lectinomics. *Biochim.Biophys.Acta* 1572:165.
2. Rabinovich, G. A., L. G. Baum, N. Tinari, R. Paganelli, C. Natoli, F. T. Liu, and S. Iacobelli. 2002. Galectins and their ligands: amplifiers, silencers or tuners of the inflammatory response? *Trends Immunol.* 23:313.
3. Cooper, D. N. and S. H. Barondes. 1999. God must love galectins; he made so many of them. *Glycobiology* 9:979.
4. Liu, F. T. 2000. Galectins: a new family of regulators of inflammation. *Clin.Immunol.* 97:79.
5. Rabinovich, G. A. 1999. Galectins: an evolutionarily conserved family of animal lectins with multifunctional properties; a trip from the gene to clinical therapy. *Cell Death. Differ.* 6:711.
6. Rabinovich, G. A., N. Rubinstein, and M. A. Toscano. 2002. Role of galectins in inflammatory and immunomodulatory processes. *Biochim.Biophys.Acta* 1572:274.
7. Gitt, M. A., E. K. Jordan, and H. Leffler. 1997. Galectin-2, Galectins-5 and -9, and Galectins-4 and -6. *Trends Glycosci. Glycotechnol.* 9:87.
8. Lahm, H., S. André, A. Hoeflich, J. R. Fischer, B. Sordat, H. Kaltner, E. Wolf, and H.-J. Gabius. 2001. Comprehensive galectin fingerprinting in a panel of 61 human tumor cell lines by RT-PCR and its implications for diagnostic and therapeutic procedures. *J. Cancer Res. Clin. Oncol.* 127:375.
9. Oka, T., S. Murakami, Y. Arata, J. Hirabayashi, K. Kasai, Y. Wada, and M. Futai. 1999. Identification and cloning of rat galectin-2: expression is predominantly in epithelial cells of the stomach. *Arch.Biochem.Biophys.* 361:195.
10. Marschal, P., V. Cannon, S. H. Barondes, and D. N. Cooper. 1994. Xenopus laevis L-14 lectin is expressed in a typical pattern in the adult, but is absent from embryonic tissues. *Glycobiology* 4:297.
11. Balomenos, D. and A. Martinez. 2000. Cell-cycle regulation in immunity, tolerance and autoimmunity. *Immunol. Today* 21:551.
12. Lenardo, M., K. M. Chan, F. Hornung, H. McFarland, R. Siegel, J. Wang, and L. Zheng. 1999. Mature T lymphocyte apoptosis--immune regulation in a dynamic and unpredictable antigenic environment. *Annu.Rev.Immunol.* 17:221-53.:221.
13. Sherr, C. J. and J. M. Roberts. 1999. CDK inhibitors: positive and negative regulators of G 1-phase progression. *Genes Dev.* 13:1501.
14. Kaltner, H. and B. Stierstorfer. 1998. Animal lectins as cell adhesion molecules. *Acta Anat (Basel)* 161:162.
15. Boudreau, N. J. and P. L. Jones. 1999. Extracellular matrix and integrin signalling: the shape of things to come. *Biochem.J.* 339:481.
16. Hemler, M. E. 1990. VLA proteins in the integrin family: structures, functions, and their role on leukocytes. *Annu.Rev.Immunol.* 8:365-400.:365.
17. Shimizu, Y., G. A. van Seventer, K. J. Horgan, and S. Shaw. 1990. Regulated expression and binding of three VLA (beta 1) integrin receptors on T cells. *Nature* 345:250.
18. Moiseeva, E. P., B. Williams, A. H. Goodall, and N. J. Samani. 2003. Galectin-1 interacts with beta-1 subunit of integrin. *Biochem. Biophys. Res. Commun.* 310:1010.
19. Brandtzaeg, P., I. N. Farstad, and G. Haraldsen. 1999. Regional specialization in the mucosal immune system: primed cells do not always home along the same track. *Immunol. Today* 20:267.
20. Furtak, V., F. Hatcher, and J. Ochieng. 2001. Galectin-3 mediates the endocytosis of beta-1 integrins by breast carcinoma cells. *Biochem.Biophys.Res.Commun.* 289:845.
21. Sturm, A., J. Itoh, J. W. Jacobberger, and C. Fiocchi. 2002. p53 negatively regulates intestinal immunity by delaying mucosal T cell cycling. *J.Clin.Invest* 109:1481.
22. Toescu, E. C. and A. Verkhratsky. 2000. Assessment of mitochondrial polarization status in living cells based on analysis of the spatial heterogeneity of rhodamine 123 fluorescence staining. *Pflugers Arch.* 440:941.
23. Scaduto, R. C., Jr. and L. W. Grotyohann. 1999. Measurement of mitochondrial membrane potential using fluorescent rhodamine derivatives. *Biophys.J.* 76:469.
24. Pace, K. E., C. Lee, P. L. Stewart, and L. G. Baum. 1999. Restricted receptor segregation into membrane microdomains occurs on human T cells during apoptosis induced by galectin-1. *J*.*Immunol 163:3801.*
25. Perillo, N. L., K. E. Pace, J. J. Seilhamer, and L. G. Baum. 1995. Apoptosis of T cells mediated by galectin-1. *Nature* 378:736.
26. Yang, R. Y., D. K. Hsu, and F. T. Liu. 1996. Expression of galectin-3 modulates T-cell growth and apoptosis. *Proc.Natl.Acad.Sci. U.S.A* 93:6737.
27. Santucci, L., S. Fiorucci, N. Rubinstein, A. Mencarelli, B. Palazzetti, B. Federici, G. A. Rabinovich, and A. Morelli. 2003. Galectin-1 suppresses experimental colitis in mice. *Gastroenterology* 124:1381.
28. Kashio, Y., K. Nakamura, M. J. Abedin, M. Seki, N. Nishi, N. Yoshida, T. Nakamura, and M. Hirashima. 2003. Galectin-9 induces apoptosis through the calcium-calpain-caspase-1 pathway. *J.Immunol.* 170:3631.
29. Hengartner, M. O. 2000. The biochemistry of apoptosis. *Nature* 407:770.
30. Kroemer, G. 2003. Mitochondrial control of apoptosis: an introduction. *Biochem. Biophys. Res. Commun.* 304:433.
31. Dias-Baruffi, M., H. Zhu, M. Cho, S. Karmakar, R. P. McEver, and R. D. Cummings. 2003. Dimeric galectin-1 induces surface exposure of phosphatidylserine and phagocytic recognition of leukocytes without inducing apoptosis. *J.Biol.Chem.* 278:41282.
32. Rabinovich, G. A., G. Daly, H. Dreja, H. Tailor, C. M. Riera, J. Hirabayashi, and Y. Chernajovsky. 1999. Recombinant galectin-1 and its genetic delivery suppress collagen-induced arthritis via T cell apoptosis. *J.Exp.Med.* 190:385.
33. Rabinovich, G. A., M. M. Iglesias, N. M. Modesti, L. F. Castagna, C. Wolfenstein-Todel, C. M. Riera, and C. E. Sotomayor. 1998. Activated rat macrophages produce a galectin-1-like protein that induces apoptosis of T cells: biochemical and functional characterization. *J.Immunol.* 160:4831.
34. Liu, X., H. Zou, C. Slaughter, and X. Wang. 1997. DFF, a heterodimeric protein that functions downstream of caspase-3 to trigger DNA fragmentation during apoptosis. *Cell* 89:175.
35. Vaux, D. L. and S. J. Korsmeyer. 1999. Cell death in development. *Cell* 96:245.
36. King, K. L. and J. A. Cidlowski. 1995. Cell cycle and apoptosis: common pathways to life and death. *J.Cell Biochem.* 58:175*.*
37. Boehme, S. A. and M. J. Lenardo. 1993. Propriocidal apoptosis of mature T lymphocytes occurs at S phase of the cell cycle. *Eur.J.Immunol.* 23:1552.
38. Vespa, G. N., L. A. Lewis, K. R. Kozak, M. Moran, J. T. Nguyen, L. G. Baum, and M. C. Miceli. 1999. Galectin-1 specifically modulates TCR signals to enhance TCR apoptosis but inhibit IL-2 production and proliferation. *J.Immunol.* 162:799.
39. Hughes, R. C. 2001. Galectins as modulators of cell adhesion. *Biochimie* 83:667.
40. Rabinovich, G. A., A. Ariel, R. Hershkoviz, J. Hirabayashi, K. I. Kasai, and O. Lider. 1999. Specific inhibition of T-cell adhesion to extracellular matrix and proinflammatory cytokine secretion by human recombinant galectin-1. *Immunology* 97:100.
41. Cooper, D. N. 1997. Galectin-1: secretion and modulation of cell interactions with laminin. *Trends Glycosci. Glycotechnol.* 9:57.
42. Fiocchi, C. 1998. Inflammatory bowel disease: etiology and pathogenesis. *Gastroenterology* 115:182.
43. Santucci, L., S. Fiorucci, F. Cammilleri, G. Servillo, B. Federici, and A. Morelli. 2000. Galectin-1 exerts immunomodulatory and protective effects on concanavalin A-induced hepatitis in mice. *Hepatology* 31:399.
44. Yang, R. Y. and F. T. Liu. 2003. Galectins in cell growth and apoptosis. *Cell Mol.Life Sci.* 60:267.
45. André, S., S. Kojima, N. Yamazaki, C. Fink, H. Kaltner, K. Kayser, and H.-J. Gabius. 1999. Galectins-1 and -3 and their ligands in tumor biology. Non-uniform properties in cell-surface presentation and modulation of adhesion to matrix glycoproteins for various tumor cell lines, in biodistribution of free and liposome-bound galectins and in their expression by breast and colorectal carcinomas with/without metastatic propensity. *J.Cancer Res. Clin. Oncol.* 125:461.
46. Targan, S. R., S. B. Hanauer, S. J. van Deventer, L. Mayer, D. H. Present, T. Braakman, K. L. DeWoody, T. F. Schaible, and P. J. Rutgeerts. 1997. A short-term study of chimeric monoclonal antibody cA2 to tumor necrosis factor alpha for Crohn's disease. Crohn's Disease cA2 Study Group. *N.EngI.J.Med.* 337:1029.
47. Elliott, M. J., R. N. Maini, M. Feldmann, J. R. Kalden, C. Antoni, J. S. Smolen, B. Leeb, F. C. Breedveld, J. D. Macfarlane, H. Bijl, and. 1994. Randomised double-blind comparison of chimeric monoclonal antibody to tumour necrosis factor alpha (cA2) versus placebo in rheumatoid arthritis. *Lancet* 344:1105.
48. Perillo, N. L., C. H. Uittenbogaart, J. T. Nguyen, and L. G. Baum. 1997. Galectin-1, an endogenous lectin produced by thymic epithelial cells, induces apoptosis of human thymocytes. *J.Exp.Med.* 185:1851.
49. Rappl, G., H. Abken, J. M. Muche, W. Sterry, W. Tilgen, S. André, H. Kaltner, S. Ugurel, H.-J. Gabius, and U. Reinhold. 2002. CD4+CD7- leukemic T cells from patients with Sezary syndrome are protected from galectin-1-triggered T cell death. *Leukemia* 16:840*.*
50. Tsuchiyama, Y., J. Wada, H. Zhang, Y. Morita, K. Hiragushi, K. Hida, K. Shikata, M. Yamamura, Y. S. Kanwar, and H. Makino. 2000. Efficacy of galectins in the amelioration of nephrotoxic serum nephritis in Wistar Kyoto rats. *Kidney Int.* 58:1941.
51. Lenardo, M. J. 1997. The molecular regulation of lymphocyte apoptosis. *Semin.Immunol.* 9:1.
52. Adams, J. M. and S. Cory. 1998. The Bcl-2 protein family: arbiters of cell survival. *Science* 281:1322.
53. Ina, K., J. Itoh, K. Fukushima, K. Kusugami, T. Yamaguchi, K. Kyokane, A. Imada, D. G. Binion, A. Musso, G. A. West, G. M. Dobrea, T. S. McCormick, E. G. Lapetina, A. D. Levine, C. A. Ottaway, and C. Fiocchi. 1999. Resistance of Crohn's disease T cells to multiple apoptotic signals is associated with a Bcl-2/Bax mucosal imbalance. *J.Immunol.* 163:1081.
54. Wesselborg, S., O. Janssens, and D. Kabelitz. 1993. Induction of activation-driven death (apoptosis) in activated but not resting peripheral blood T cells. *J.Immunol.* 150:4338.
55. Inohara, H., S. Akahani, and A. Raz. 1998. Galectin-3 stimulates cell proliferation. *Exp. Cell Res.* 245:294.
56. Sramkoski, R. M., S. W. Wormsley, W. E. Bolton, D. C. Crumpler, and J. W. Jacobberger. 1999. Simultaneous detection of cyclin B1, p105, and DNA content provides complete cell cycle phase fraction analysis of cells that endoreduplicate. *Cytometry* 35:274.
57. Springer, T. A. 1990. Adhesion receptors of the immune system. *Nature* 346:425.
58. Springer, T. A. 1994. Traffic signals for lymphocyte recirculation and leukocyte emigration: the multistep paradigm. *Cell* 76:301.
59. Schwartz, M. A., M. D. Schaller, and M. H. Ginsberg. 1995. Integrins: emerging paradigms of signal transduction. *Annu.Rev.Cell Dev.Biol.* 11:549-99.:549.
60. Dailey, M. O. 1998. Expression of T lymphocyte adhesion molecules: regulation during antigen-induced T cell activation and differentiation. *Crit Rev.Immunol.* 18:153.
61. Zell, T., W. J. Kivens, S. A. Kellermann, and Y. Shimizu. 1999. Regulation of integrin function by T cell activation: points of convergence and divergence. *Immunol. Res.* 20:127.
62. Brewer, C. F. 2002. Binding and cross-linking properties of galectins. *Biochim.Biophys.Acta* 1572:255.
63. Kuwabara, I. and F. T. Liu. 1996. Galectin-3 promotes adhesion of human neutrophils to laminin. *J.Immunol.* 156:3939.
64. Ochieng, J., M. L. Leite-Browning, and P. Warfield. 1998. Regulation of cellular adhesion to extracellular matrix proteins by galectin-3. *Biochem. Biophys. Res. Commun.* 246:788.

## Claims

1. Use of galectin-2 or of a nucleic acid coding for galectin-2 or of its complementary strand, or of a nucleic acid hybridizing to such coding nucleic acid or its complementary strand, for the manufacture of a medicament for the treatment of a patient having a disease with impaired apoptosis of T-cells, wherein said disease with impaired apoptosis of T-cells is selected from the group comprising autoimmune diseases and malignant T-cell diseases.

2. Use according to claim 1, wherein said impaired apoptosis, in particular said impaired apoptosis of T-cells is involved in or associated with the pathogenesis of said disease.

3. Use according to any of the foregoing claims, wherein said autoimmune diseases are selected from the group comprising rheumatoid arthritis, inflammatory bowel diseases, multiple sclerosis, psoriasis, lupus erythematodes, scleroderma, autoimmune hepatitis and autoimmune nephritis.

4. Use according to any of the foregoing claims, wherein said malignant T-cell diseases are selected from the group comprising peripheral and lymphoblastic/nodal and extranodal T-non-Hodgkin-lymphomas

5. Use according to claim 3, wherein said inflammatory bowel diseases are Crohn's disease or colitis ulcerosa or indeterminate colitis.

6. Use according to claim 5, wherein said inflammatory bowel disease is Crohn's disease.

7. Use according to claim 3, wherein said autoimmune disease is rheumatoid arthritis.

8. Use according to any of the foregoing claims, wherein said galectin-2 is human or rat galectin-2.

9. Use according to any of the foregoing claims, wherein said galectin-2 has an amino acid sequence selected from the group comprising SEQ ID NO:1 and SEQ ID NO: 2.

10. Use according to any of the foregoing claims, wherein said galectin-2 is administered in combination with an agent suppressing T-cell proliferation and/or an agent inducing T-cell apoptosis.

11. Use according to claim 10, wherein said agent suppressing T-cell proliferation is selected from the group comprising steroids, macrolides, such as cyclosporin and rapamycin, tacrolimus, azathioprine, 6-mercaptopurine, methotrexate and cyclophosphamide.

12. Use according to claim 10, wherein said T-cell apoptosis inducing agent is selected from the group comprising anti-TNFα-antibody (infliximab, adalimumab and CDP 870), etanercept, leflunamide, natalizumab (anti-Integrin α4β7 mAb), visilizumab (anti-CD3 mAb).

13. Use according to any of the foregoing claims, wherein said galectin-2 is administered in combination with a drug that induces T-cell apoptosis via a caspase-8 dependent pathway, or wherein said galectin-2 is administered in a patient failing or having failed to show a measurable response to a drug which is known to normally induce T-cell apoptosis via a caspase-8 dependent pathway.

14. Use according to any of the foregoing claims, wherein said galectin-2 is administered in combination with anti-inflammatory drugs such as 5-Aminosalicylates (5-ASA), mesalazine, olsalazin, balsalazin, sulfapyridin and non-steroidal anti-inflammatory agent and/or an antirheumatic agent.

15. Use according to claim 14, wherein said antirheumatic agent is a disease modifying anti rheumatic drug (DMARD).

16. Use according to claim 15, wherein said disease modifying anti-rheumatic drug is selected from the group comprising aspirin, naproxen, diclofenac, ibuprofen, naprosyn, indomethacin, piroxican and biological drugs selected from the group comprising anakinra and etodolac.

17. Use according to claim 15, wherein said antirheumatic agent is selected from the group comprising gold compounds, D-penicillamin, antimalaria drugs such as chloroquin, and sulfasalazine.

18. Use according to any of the foregoing claims, wherein said galectin-2 is administered in combination with cyclo-oxygenase-2-inhibitors (COX-2-inhibitors).

19. Use according to claim 18, wherein said cyclo-oxygenase-2-inhibitors are selected from the group comprising celecoxib, rofecoxib, and valdecoxib.

20. Use according to any of the foregoing claims, wherein said galectin-2 is administered in combination with a T-cell activating agent.

21. Use according to any of the foregoing claims, wherein said galectin-2 is administered in combination with a β-galactoside.

22. Use according to claim 21, wherein said β-galactoside is lactose.

23. Use according to any of the foregoing claims, wherein said galectin-2 is administered by systemical administration and/ or topical administration.

24. Use according to claim 23, wherein said administration occurs by ingestion, preferably orally or anally, and/or by injection, preferably by intravenous, intramuscular, intraperitoneal or subcutaneous injection, and/or by nasal application.

25. Use according to any of claims 23 - 24, wherein said galectin-2 is administered as enema and/or as suppository and/or as delayed release dosage form, e. g. encapsulated in a pH dependent release matrix.

26. Use according to any of claims 23 - 25, wherein said galectin-2 is administered in a pegylated or non-pegylated form or as a mixture of the two forms.

27. Use according to any of the foregoing claims, wherein said patient is one having a pathological condition in which, before administration of galectin-2, a subset of the patient's T-cells and/or a subset of the patient's macrophages and/or a subset of the patient's antigen-presenting-cells fail to undergo apoptosis, preferably adequate apoptosis or wherein a subset of the patient's T-cells and/or macrophages and/or antigen-presenting cells show an impaired or defective apoptosis.

28. Use according to claim 27, wherein said subset of T-cells are T-cells that have previously been activated, preferably via the CD3-pathway or the CD2-pathway or via mitogens, co-stimulatory molecules such as CD28 or CD40, or other pathways such as Toll-like receptors or integrins.

29. Use according to any of claims 27 - 28, wherein said subset of T-cells and/or macrophages and/or antigen-presenting cells are not resting.

30. Use according to claim 29, wherein said subset of T-cells and/or macrophages and/or antigen-presenting cells are not cells that have exited from the cell cycle or are not cells that are arrested in any phase of the cell-cycle.

31. Use according to any of claims 27 - 30, wherein said subset of T-cells and/or macrophages and/or antigen-presenting cells is primarily located in said patient's joints, preferably synovial joints, and/or in said patient's gastrointestinal tract, preferably the lining of said gastrointestinal tract, and/or in said patient's skin, and/or lung and/or liver and/or kidney and/or are a population of peripheral blood cells which are recruited in a mucosa during inflammation.

32. Use according to any of the foregoing claims, wherein said patient is one having a pathological condition in which, before administration of galectin-2, the ratio between Bcl-2-protein and Bax-protein in T-cells is disbalanced in favour of the anti-apoptotic Bcl-2.

## Patentansprüche

1. Verwendung von Galectin-2 oder einer Nukleinsäure, die für Galectin-2 kodiert, oder von ihrem komplementären Strang oder von einer Nukleinsäure, die an eine solche kodierende Nukleinsäure oder an ihren komplementären Strang hybridisiert, zur Herstellung eines Medikaments zur Behandlung eines Patienten mit einer Krankheit mit beeinträchtigter Apoptose von T-Zellen, wobei die Krankheit mit beeinträchtigter Apoptose von T-Zellen ausgewählt ist aus der Gruppe, umfassend Autoimmunkrankheiten und maligne T-Zell-Krankheiten.

2. Verwendung nach Anspruch 1, wobei die beeinträchtigte Apoptose, insbesondere die beeinträchtigte Apoptose von T-Zellen an der Pathogenese der Krankheit beteiligt oder damit assoziiert ist.

3. Verwendung nach einem der vorangehenden Ansprüche, wobei die Autoimmunkrankheiten ausgewählt sind aus der Gruppe, umfassend rheumatoide Arthritis, entzündliche Darmerkrankungen, Multiple Sklerose, Psoriasis, Lupus erythematodes, Skleroderma, Autoimmunhepatitis und Autoimmunnephritis.

4. Verwendung nach einem der vorangehenden Ansprüche, wobei die malignen T-Zell-Krankheiten ausgewählt sind aus der Gruppe, umfassend periphere und lymphoblastische/nodale und extranodale T-nicht-Hodgkin-Lymphome.

5. Verwendung nach Anspruch 3, wobei die entzündlichen Darmerkrankungen Morbus Crohn oder Colitis Ulcerosa oder unbestimmte Colitis sind.

6. Verwendung nach Anspruch 5, wobei die entzündliche Darmkrankheit Morbus Crohn ist.

7. Verwendung nach Anspruch 3, wobei die Autoimmunkrankheit rheumatoide Arthritis ist.

8. Verwendung nach einem der vorangehenden Ansprüche, wobei Galectin-2 humanes oder Ratten-Galectin-2 ist.

9. Verwendung nach einem der vorangehenden Ansprüche, wobei das Galectin-2 eine Aminosäuresequenz hat, ausgewählt aus der Gruppe, umfassend SEQ ID NO: 1 und SEQ ID NO: 2.

10. Verwendung nach einem der vorangehenden Ansprüche, wobei das Galectin-2 in Kombination mit einem Mittel, das T-Zellproliferation unterdrückt, und/oder einem Mittel verabreicht wird, das T-Zellapoptose induziert.

11. Verwendung nach Anspruch 10, wobei das Mittel, das T-Zellproliferation unterdrückt, ausgewählt ist aus der Gruppe, umfassend Steroide, Macrolide, wie etwa Cyclosporin und Rapamycin, Tacrolimus, Azathioprin, 6-Mercaptopurin, Methotrexat und Cyclophosphamid.

12. Verwendung nach Anspruch 10, wobei das T-Zellapoptose-induzierende Mittel ausgewählt ist aus der Gruppe, umfassend Anti-TNFα-Antikörper (Infliximab, Adalimumab und CDP 870), Etanercept, Leflunamid, Natalizumab (Anti-Integrin α4β7 mAb), Visilizumab (Anti-CD3 mAb).

13. Verwendung nach einem der vorangehenden Ansprüche, wobei das Galectin-2 in Kombination mit einer Arznei verabreicht wird, die T-Zell-Apoptose über einen Caspase-8-abhängigen Weg induziert, oder wobei das Galectin-2 einem Patienten verabreicht wird, der keine meßbare Antwort auf eine Arznei zeigt oder gezeigt hat, von der bekannt ist, daß sie normalerweise T-Zell-Apoptose über einen Caspase-8-abhängigen Weg induziert.

14. Verwendung nach einem der vorangehenden Ansprüche, wobei das Galectin-2 in Kombination mit anti-entzündlichen Arzneien verabreicht wird, wie etwa 5-Aminosalicylate (5-ASA), Mesalazin, Olsalazin, Balsalazin, Sulfapyridin und einem nicht-steroid-anti-entzündliches Mittel und/oder einem antirheumatischen Mittel.

15. Verwendung nach Anspruch 14, wobei das antirheumatische Mittel eine krankheitsmodifizierende anti-rheumatische Arznei (DMARD) ist.

16. Verwendung nach Anspruch 15, wobei die krankheitsmodifizierende anti-rheumatische Arznei ausgewählt ist aus der Gruppe, umfassend Aspirin, Naproxen, Diclofenac, Ibuprofen, Naprosyn, Indomethacin, Piroxican und biologische Arzneien, ausgewählt aus der Gruppe, umfassend Anakinra und Etodolac.

17. Verwendung nach Anspruch 15, wobei das anti-rheumatische Mittel ausgewählt ist aus der Gruppe, umfassend Goldverbindungen, D-Penicillamin, anti-Malaria Arzneien, wie etwa Chlorochin, und Sulfasalazin.

18. Verwendung nach einem der vorangehenden Ansprüche, wobei das Galectin-2 in Kombination mit Cyclo-Oxygenase-2-Inhibitoren (COX-2 Inhibitoren) verabreicht wird.

19. Verwendung nach Anspruch 18, wobei die Cyclo-Oxygenase-2-Inhibitoren ausgewählt sind aus der Gruppe, umfassend Celecoxib, Rofecoxib, und Valdecoxib.

20. Verwendung nach einem der vorangehenden Ansprüche, wobei das Galectin-2 in Kombination mit einem T-Zell-aktivierenden Mittel verabreicht wird.

21. Verwendung nach einem der vorangehenden Ansprüche, wobei das Galectin-2 in Kombination mit einem β-Galactosid verabreicht wird.

22. Verwendung nach Anspruch 21, wobei das β-Galactosid Lactose ist.

23. Verwendung nach einem der vorangehenden Ansprüche, wobei das Galectin-2 über systemische Verabreichung und/oder topische Verabreichung verabreicht wird.

24. Verwendung nach Anspruch 23, wobei die Verabreichung mittels Aufnahme, bevorzugt oral oder anal, und/oder mittels Injektion, bevorzugt mittels intravenöser, intramuskulärer, intraperitonealer oder subkutaner Injektion, und/oder mittels nasaler Anwendung erfolgt.

25. Verwendung nach einem der Ansprüche 23 - 24, wobei das Galectin-2 als Einlauf ("Enema") und/oder als Zäpfchen und/oder als Dosierungsform mit verzögerter Freisetzung verabreicht wird, z.B. in einer pH-abhängigen Freisetzungsmatrix verkapselt.

26. Verwendung nach einem der Ansprüche 23 - 25, wobei das Galectin-2 in einer pegylierten oder nicht-pegylierten Form oder als eine Mischung der beiden Formen verabreicht wird.

27. Verwendung nach einem der vorangehenden Ansprüche, wobei der Patient ein Patient mit einem pathologischen Zustand ist, in dem vor der Verabreichung von Galectin-2 eine Teilmenge der T-Zellen des Patienten und/oder eine Teilmenge der Makrophagen des Patienten und/oder eine Teilmenge der Antigen-präsentierenden Zellen des Patienten keine Apoptose durchlaufen, bevorzugt adäquate Apoptose, oder wobei eine Teilmenge der T-Zellen und/oder Makrophagen und/oder Antigen-präsentierenden Zellen des Patienten beeinträchtigte oder fehlerhafte Apoptose zeigen.

28. Verwendung nach Anspruch 27, wobei die Teilmenge von T-Zellen T-Zellen sind, die zuvor aktiviert worden sind, bevorzugt über den CD3-Weg oder den CD2-Weg oder über Mitogene, co-stimulatorische Moleküle, wie etwa CD28 oder CD40, oder andere Wege, wie etwa Toll-artige Rezeptoren oder Integrine.

29. Verwendung nach einem der Ansprüche 27 - 28, wobei die Teilmenge von T-Zellen und/oder Makrophagen und/oder Antigen-präsentierenden Zellen nicht ruhend sind.

30. Verwendung nach Anspruch 29, wobei die Teilmenge von T-Zellen und/oder Makrophagen und/oder Antigen-präsentierenden Zellen keine Zellen sind, die aus dem Zellzyklus ausgeschieden sind, oder keine Zellen sind, die in irgendeiner Phase des Zellzyklus arretiert sind.

31. Verwendung nach einem der Ansprüche 27 - 30, wobei die Teilmenge von T-Zellen und/oder Makrophagen und/oder Antigen-präsentierenden Zellen sich hauptsächlich in den Gelenken des Patienten befinden, bevorzugt Synovialgelenken, und/oder in dem Gastrointestinaltrakt des Patienten, bevorzugt der Auskleidung des Gastrointestinaltrakts, und/oder in der Haut des Patienten und/oder der Lunge und/oder der Leber und/oder der Niere, und/oder eine Population von Peripherblutzellen sind, die in eine Schleimhaut während einer Entzündung rekrutiert werden.

32. Verwendung nach einem der vorangehenden Ansprüche, wobei der Patient ein Patient mit einem pathologischen Zustand ist, in dem vor der Verabreichung von Galectin-2 das Verhältnis zwischen Bcl-2-Protein und Bax-Protein in T-Zellen zu Gunsten des Anti-Apoptose-Bcl-2 verschoben ist.

## Revendications

1. Utilisation de galectine 2 ou d'un acide nucléique codant la galectine 2 ou son brin complémentaire, ou d'un acide nucléique hybridant avec un tel acide nucléique codant ou son brin complémentaire, pour la fabrication d'un médicament pour le traitement d'un patient ayant une maladie avec une apoptose dérégulée des cellules T, dans laquelle ladite maladie avec une apoptose dérégulée des cellules T est choisie parmi le groupe comprenant les maladies auto-immunes et les maladies malignes des cellules T.

2. Utilisation selon la revendication 1, dans laquelle ladite apoptose dérégulée, en particulier ladite apoptose dérégulée des cellules T est englobée dans ou associée à la pathogenèse de ladite maladie.

3. Utilisation selon l'une quelconque des revendications précédentes, dans laquelle lesdites maladies auto-immunes sont choisies parmi le groupe comprenant l'arthrite rhumatoïde, les maladies inflammatoires de l'intestin, la sclérose en plaques, le psoriasis, le lupus érythémateux, la sclérodermie, l'hépatite auto-immune et la néphrite auto-immune.

4. Utilisation selon l'une quelconque des revendications précédentes, dans laquelle lesdites maladies malignes des cellules T sont choisies parmi le groupe comprenant les lymphomes T non hodgkiniens périphériques et lymphoblastiques/nodulaires et extranodulaires.

5. Utilisation selon la revendication 3, dans laquelle lesdites maladies inflammatoires de l'intestin sont la maladie de Crohn ou la colite ulcéreuse ou une colite indéterminée.

6. Utilisation selon la revendication 5, dans laquelle ladite maladie inflammatoire de l'intestin est la maladie de Crohn.

7. Utilisation selon la revendication 3, dans laquelle la maladie auto-immune est l'arthrite rhumatoïde.

8. Utilisation selon l'une quelconque des revendications précédentes, dans laquelle ladite galectine 2 est une galectine 2 humaine ou de rat.

9. Utilisation selon l'une quelconque des revendications précédentes, dans laquelle ladite galectine 2 a une séquence des acides aminés choisie parmi le groupe comprenant la SEQ ID NO:1 et la SEQ ID NO:2.

10. Utilisation selon l'une quelconque des revendications précédentes, dans laquelle ladite galectine 2 est administrée en combinaison avec un agent supprimant la prolifération des cellules T et/ou un agent induisant l'apoptose des cellules T.

11. Utilisation selon la revendication 10, dans laquelle l'agent supprimant la prolifération des cellules T est choisi parmi le groupe comprenant des stéroïdes, des macrolides, comme la cyclosporine et la rapamycine, le tacrolimus, l'azathioprine, la 6-mercaptopurine, le méthotrexate et le cyclophosphamide.

12. Utilisation selon la revendication 10, dans laquelle ledit agent induisant l'apoptose des cellules T est choisi parmi le groupe comprenant l'anticorps anti-TNFα (infliximab, adalimumab et CDP 870), l'étanercept, la leflunamide, le natalizumab (anti-intégrine α4β7 mAB), le visilizumab (anti-CD3 mAB).

13. Utilisation selon l'une quelconque des revendications précédentes, dans laquelle ladite galectine 2 est administrée en combinaison avec un médicament qui induit l'apoptose des cellules T via une voie qui dépend de la caspase-8 ou dans laquelle ladite galectine 2 est administrée à un patient échouant ou ayant échoué à montrer une réponse mesurable à un médicament qui est connu pour induire normalement l'apoptose des cellules T via une voie dépendante de la caspase-8.

14. Utilisation selon l'une quelconque des revendications précédentes, dans laquelle ladite galectine 2 est administrée en combinaison avec des médicaments anti-inflammatoires comme des 5-aminosalicylates (5-ASA), la mesalazine, l'olsalazine, la balsalazine, la sulfapyridine et un agent anti-inflammatoire non stéroïdien et/ou un agent antirhumatismal.

15. Utilisation selon la revendication 14, dans laquelle ledit agent antirhumatismal est un médicament antirhumatismal modifiant la maladie (DMARD).

16. Utilisation selon la revendication 15, dans laquelle ledit médicament antirhumatismal modifiant la maladie est choisi parmi le groupe comprenant l'aspirine, le naproxen, le diclofénac, l'ibuprofen, le naprosyn, l'indométacine, le piroxican et des médicaments biologiques choisis parmi le groupe comprenant l'anakinra et l'étodolac.

17. Utilisation selon la revendication 15, dans laquelle ledit agent antirhumatismal est choisi parmi le groupe comprenant des composés de l'or, la D-pénicillamine, des médicaments antimalariques comme la chloroquine et la sulfasalazine.

18. Utilisation selon l'une quelconque des revendications précédentes, dans laquelle ladite galectine 2 est administrée en combinaison avec des inhibiteurs de la cyclo-oxygénase-2 (COX-2-inhibiteurs).

19. Utilisation selon la revendication 18, dans laquelle lesdits inhibiteurs de la cyclo-oxygénase-2 sont choisis parmi le groupe comprenant le celecoxib, le rofecoxib et le valdecoxib.

20. Utilisation selon l'une quelconque des revendications précédentes, dans laquelle ladite galectine 2 est administrée en combinaison avec un agent d'activation des cellules T.

21. Utilisation selon la revendication 20, dans laquelle ladite galectine 2 est administrée en combinaison avec un β-galactoside.

22. Utilisation selon la revendication 21, dans laquelle ledit β-galactoside est le lactose.

23. Utilisation selon l'une quelconque des revendications précédentes, dans laquelle ladite galectine 2 est administrée par administration systémique et/ou administration topique.

24. Utilisation selon la revendication 23, dans laquelle ladite administration apparaît par ingestion, de préférence par voie orale ou anale, et/ou par injection, de préférence par injection intraveineuse, intramusculaire, intrapéritonéale ou sous-cutanée, et/ou par application nasale.

25. Utilisation selon l'une quelconque des revendications 23-24, dans laquelle ladite galectine 2 est administrée comme énéma et/ou suppositoire et/ou forme posologique à libération retardée, par exemple encapsulée dans une matrice de libération dépendante du pH.

26. Utilisation selon l'une quelconque des revendications 23-25, dans laquelle ladite galectine 2 est administrée dans une forme pégylée ou non pégylée ou sous forme d'un mélange des deux formes.

27. Utilisation selon l'une quelconque des revendications précédentes, dans laquelle ledit patient est un patient ayant une affection pathologique dans laquelle, avant l'administration de galectine 2, un sous-ensemble des cellules T du patient et/ou un sous-ensemble des macrophages du patient et/ou un sous-ensemble des cellules présentant l'antigène du patient échoue à subir l'apoptose, de préférence l'apoptose adéquate ou dans laquelle un sous-ensemble des cellules T du patient et/ou des macrophages et/ou des cellules présentant l'antigène montre une apoptose dérégulée ou déficiente.

28. Utilisation selon la revendication 27, dans laquelle ledit sous-ensemble des cellules T est constitué de cellules T qui ont auparavant été activées, de préférence via la voie du CD3 ou la voie du CD2 ou via des mitogènes, des molécules costimulantes comme CD28 ou CD40, ou d'autres voies comme des récepteurs de type Toll ou des intégrines.

29. Utilisation selon l'une quelconque des revendications 27-28, dans laquelle ledit sous-ensemble des cellules T et/ou des macrophages et/ou des cellules présentant l'antigène n'est pas au repos.

30. Utilisation selon la revendication 29, dans laquelle ledit sous-ensemble des cellules T et/ou des macrophages et/ou des cellules présentant l'antigène n'est pas constitué de cellules qui ont été sorties du cycle cellulaire ou n'est pas constitué de cellules qui ont été arrêtées dans une phase quelconque du cycle cellulaire.

31. Utilisation selon l'une quelconque des revendications 27-30, dans laquelle ledit sous-ensemble des cellules T et/ou des macrophages et/ou des cellules présentant l'antigène est principalement localisé dans lesdites articulations du patient, de préférence les articulations synoviales et/ou dans ledit tractus gastro-intestinal du patient, de préférence le revêtement intérieur dudit tractus gastro-intestinal et/ou dans ladite peau du patient et/ou le poumon et/ou le foie et/ou le rein et/ou est une population de cellules du sang périphérique qui sont recrutées dans une muqueuse durant l'inflammation.

32. Utilisation selon l'une quelconque des revendications précédentes, dans laquelle ledit patient est un patient ayant une affection pathologique dans laquelle, avant administration de galectine 2, le rapport entre la Bcl-2-protéine et la Bax-protéine dans les cellules T est déséquilibré en faveur de la Bcl-2 anti-apoptotique.
